(19) Europäisches Patentamt
European Patent Office
Office européen des brevets

(11) Numéro de publication: **0 126 094**
**B1**

(12) # FASCICULE DE BREVET EUROPEEN

(45) Date de publication du fascicule du brevet: **20.06.90**

(51) Int. Cl.$^5$: **C 07 D 211/90,** C 07 D 401/12, C 07 D 405/12, C 07 D 409/04, A 61 K 31/455

(21) Numéro de dépôt: **83903371.9**

(22) Date de dépôt: **18.11.83**

(86) Numéro de dépôt international: **PCT/CH83/00128**

(87) Numéro de publication internationale: **WO 84/02132 07.06.84 Gazette 84/14**

(54) **NOUVEAUX ESTERS DE 1,4-DIHYDROPYRIDINES ET LEURS PROCEDES DE PREPARATION ET MEDICAMENTS CONTENANT LESDITS ESTERS.**

(30) Priorité: **24.11.82 CH 6858/82**

(43) Date de publication de la demande: **28.11.84 Bulletin 84/48**

(45) Mention de la délivrance du brevet: **20.06.90 Bulletin 90/25**

(84) Etats contractants désignés: **AT BE CH DE FR GB LI LU NL SE**

(56) Documents cités:
**EP-A-0 007 293**
**EP-A-0 012 180**
**EP-A-0 052 300**
**EP-A-0 088 276**
**DE-A-2 117 573**
**FR-A-2 132 830**
**FR-A-2 182 983**
**FR-A-2 302 093**
**FR-A-2 405 937**
**US-A-3 574 843**
**US-A-4 258 042**

**Le dossier contient des informations techniques présentées postérieurement au dépôt de la demande et ne figurant pas dans le présent fascicule.**

(73) Titulaire: **CERMOL S.A.**
**CH-1902 Evionnaz (CH)**

(72) Inventeur: **SUNKEL LETELIER, Carlos**
**Mateo Inurria 30**
**E-Madrid 16 (ES)**
Inventeur: **FAU DE CASA-JUANA MUNOZ, Miguel**
**Mateo Inurria 30**
**E-Madrid 16 (ES)**
Inventeur: **STATKOV, Peter, R.**
**6, rue du Roveray**
**CH-1207 Genève (CH)**
Inventeur: **STRAUMANN, Danielle**
**6, rue Pré Borvey**
**CH-1920 Martigny (CH)**

(74) Mandataire: **Vuille, Roman et al**
**c/o KIRKER & Cie S.A. 14, rue du Mont-Blanc**
**Case Postale 872**
**CH-1211 Genève 1 (CH)**

(56) Documents cités:
**Derwent Japanese Patents vol. 7, no. 100, 28 avril 1983 Derwent Patent Publications, section Chemical, C-164(1245) JP,A,5826882, Tanabe, 17 février 1983**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**EP 0 126 094 B1**

Courier Press, Leamington Spa, England.

## Description

La présente invention concerne des nouvelles 1,4-dihydropyridines, des procédés permettant de les obtenir, et des médicaments qui les contiennent, de préférence comme agents exerçant un effet sur les vaisseaux, notamment comme agents coronaires ou agents anti-athéromatiques. L'invention a plus particulièrement pour objet de nouveaux esters de 1,4-dihydropyridine, ainsi que leurs sels, que l'on peut représenter au moyen de la formule:

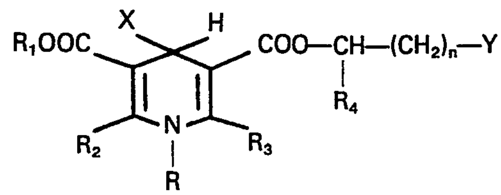

(I)

dans laquelle:

R désigne un atome d'hydrogène, ou un radical alkyle inférieur;

$R_2$ et $R_3$ représentent chacun un atome d'hydrogène ou un radical alkyle inférieur à chaîne droite ou ramifiée;

$R_4$ désigne un atome d'hydrogène ou un radical alkyle inférieur à chaîne droite,

*n* est un nombre égal à 0, 1, 2 ou 3;

X désigne un radical phényle qui porte, le cas échéant, un à trois substituants nitro, alkyle ou alkoxy inférieur, ou halogène;

$R_1$ désigne un radical alkyle ou alcényle, à chaîne droite ou ramifiée, contenant de 1 à 6 atomes de carbone et pouvant être interrompue par un ou deux atomes d'oxygène ou de soufre, ou un groupe alicyclique substitué ou non, ou le groupe

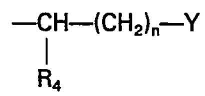

à l'exception du cas où Y représente le radical 2-tétrahydrofurfuryle; et

Y désigne le radical dérivé des amides N-substituées des acides nicotinique, salicylique et 4-hydroxybenzoïque ou des pipérazines monoacylées ou sulfonylées N-substituées de formule:

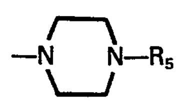

dans laquelle $R_5$ désigne un radical acyle ou arylsulfonyle, ou un groupe de formule $-OCO-R_6$ ou $-OR_6$ dans laquelle $R_6$ désigne un radical acylamino, ou encore un groupe 2-tétrahydrofurfuryle ou N-(4-benzoylpipéridinyle).

De nombreux dérivés de 1,4-dihydropyridine ont déjà été décrits dans la littérature scientifique. En particulier, les brevets US—A—3.574.843, et FR—A—2.132.830, EP—A—0012.180, FR—A—2.302.903 et FR—2.182.983 décrivent des 1,4-dihydropyridines diversement substituées, notamment par un groupe aromatique en position 4: plusieurs de ces composés ont été proposés pour la préparation de médicaments, en particulier de médicaments destinés au traitement d'affections coronariennes. Certains d'entre eux sont même commercialisés depuis quelque temps.

Les nouveaux esters de 1,4-dihydropyridine (I), objets de l'invention, se distinguent par une activité pharmacologique remarquable, au niveau des vaisseaux sanguins, en particulier au niveau des vaisseaux coronaires.

Comparés à des produits similaires connus lors de tests sur animaux, les composés de formule (I) se sont révélés, à dose thérapeutique égale, nettement moins toxiques et sensiblement plus actifs. Ils peuvent être de ce fait advantageusement proposés pour le traitement préventif ou curatif de maladies vasculaires, notamment cardio-vasculaires.

Les composés (I), peuvent être obtenus selon les schémas de réaction suivants:

a) en faisant réagir des esters d'acides β-cétocarboxyliques de formule (II):

$$R_2-CO-CH_2-COOR_1 \qquad (II)$$

(dans laquelle $R_1$ et $R_2$ sont définis ci-dessus) avec des amines de formule (III):

$$R-NH_2 \qquad (III)$$

(dans laquelle R est définis ci-dessus) le cas échéant après isolement des énamines éventuellement produites à partir des esters d'acides β-cétocarboxyliques et des amines, de formule (IV):

$$\begin{array}{l} \quad\;\; NH{-}R \\ \quad\;\;\; | \\ R_2{-}C{=}CH{-}COOR_1 \end{array} \qquad (IV)$$

(dans laquelle R, $R_1$ et $R_2$ sont définis ci-dessus) avec les dérivés ylidéniques de formule (V):

$$\begin{array}{l} \qquad\qquad\qquad\; R_4 \\ \qquad\qquad\qquad\;\; | \\ \qquad\quad COO{-}CH{-}(CH_2)_n{-}Y \\ \qquad\quad\;\; | \\ X{-}CH{=}C{-}CO{-}R_3 \end{array} \qquad (V)$$

(dans laquelle X, $R_3$, $R_4$, n et Y sont définis ci-dessus), obtenus par réaction des aldéhydes de formule (VI):

$$X{-}CHO \qquad (VI)$$

(dans laquelle X est défini ci-dessus), avec les esters d'acides β-cétocarboxyliques de formule (VII):

$$\begin{array}{l} R_3{-}CO{-}CH_2{-}COO{-}CH{-}(CH_2)_n{-}Y \\ \qquad\qquad\qquad\qquad\quad\; | \\ \qquad\qquad\qquad\qquad\quad R_4 \end{array} \qquad (VII)$$

(dans laquelle $R_3$, $R_4$, n et Y sont définis ci-dessus); ou

b) en faisant réagir des esters d'acides β-cétocarboxyliques de formule (VII) avec des amines (III), le cas échéant après isolement des énamines éventuellement formées à partir des esters, d'acides β-cétocarboxyliques et des amines, de formule (VIII):

$$\begin{array}{l} \quad\;\; NH{-}R \\ \quad\;\;\; | \\ R_3{-}C{=}CH{-}COO{-}CH{-}(CH_2)_n{-}Y \\ \qquad\qquad\qquad\qquad\; | \\ \qquad\qquad\qquad\qquad R_4 \end{array} \qquad (VIII)$$

(dans laquelle R, $R_3$, $R_4$, n et Y ont les définitions données ci-dessus) avec des dérivés ylidéniques de formule (IX):

$$\begin{array}{l} \qquad\quad COOR_1 \\ \qquad\quad\;\; | \\ X{-}CH{=}C{-}CO{-}R_2 \end{array} \qquad (IX)$$

(dans laquelle X, $R_1$ et $R_2$ sont définis ci-dessus) obtenus par réaction des aldéhydes de formule (VI) avec les esters d'acides β-cétocarboxyliques de formule (II); ou

c) en faisant réagir des esters d'acides β-cétocarboxyliques de formule (II) avec des énamines de formule (VIII) et des aldéhydes de formule (VI); ou

d) en faisant réagir des esters d'acides β-cétocarboxyliques de formule (VII), avec des énamines de formule (IV) et avec des aldéhydes de formule (VI); ou

e) en faisant réagir deux moles d'esters d'acides β-cétocarboxyliques de formule (VII), avec une mole d'amine de formule (III) et une mole d'un aldéhyde de formule (VI).

Le cas échéant, avec un acide ou peut préparer les sels des composés obtenus dans les variantes a) à e) du procédé. Dans la formule (I), le reste R désigne de préférence un reste alkyle à chaîne droite ou ramifiée en $C_1$ à $C_4$, tel que méthyle, éthyle, n-propyle, isopropyle, n-butyle et tertio-butyle.

Dans la formule (I), le reste $R_1$ désigne de préférence un reste alkyle à chaîne droite ou ramifiée en $C_1$ à $C_4$, tel que méthyle, éthyle, n-propyle, isopropyle, n-butyle, isobutyle et tertiobutyle ou un reste alcényle à chaîne droite ou ramifiée en $C_2$ à $C_4$, tel que éthényle, propényle-1 ou 2 et butényle. $R_1$ peut aussi désigner un reste cycloalkylique ayant de préférence 5 ou 6 atomes de carbone, tel que cyclopentyle, cyclohexyle et 3,3,5-triméthylcyclohexyle. Quand un atome d'oxygène ou de soufre existe dans la chaîne hydrocarbonée, $R_1$ désigne de préférence des restes de formules —W—O—Z et —W—S—Z, dans laquelle W désigne un groupe alkylénique à chaîne droite ou ramifiée en $C_1$ à $C_3$, tel que méthylène, éthylène et isopropylène et isopropylène, et Z désigne un radical alkyle à chaîne droite ou ramifiée en $C_1$ à $C_4$ tel que méthyle, éthyle, n-propyle, isopropyle, n-butyle et tertio-butyle. Aussi $R_1$ désigne le radical cyclique tétrahydrofurfurylique-2.

Dans la formule (I), les groupes alkyle $R_2$ et $R_3$ désignent de préférence un radical alkyle linéaire ou en $C_1$ à $C_4$ tel que méthyle, éthyle, n-propyle, isopropyle et tertiobutyle.

Dans la formule (I), le groupe alkyle $R_4$ désigne de préférence un atome d'hydrogène ou un radical alkyle à chaîne droite en $C_1$ à $C_2$, tel que méthyle et éthyle.

Dans la formule (I), le reste X désigne un radical phényle, substitué ou non. Le reste X peut porter un ou plusieurs, de préférence 1 à 3 et notamment 1 ou 2 substituants identiques ou différents. Ces substituants sont, par exemple, des groupes alkyle tels que méthyle, éthyle, n-propyle, isopropyle, n-butyle, isobutyle et tertio-butyle; des groupes alkoxy tels que méthoxy, éthoxy, n-propyloxy et isopropyloxy; des halogènes, de préférence fluor, chlore, brome et iode; des groupes nitro.

Dans la formule (I), le groupe Y désigne de préférence les amides N-substitués des acides nicotinique, salicylique et 4-hydroxybenzoique et des pipérazines, monoacrylées et sulfonylées de formule:

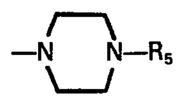

dans laquelle $R_5$ désigne un radical acyle tel qu'acétyle, 2-furoyle, 2-thiophénecarbonyle et cinnamoyle. Aussi $R_5$ désigne un radical arylsulfonyle tel que benzènesulfonyle; ces derniers radicaux aromatiques peuvent être substitués à leur tour par un ou plusieurs groupes alkyle tel que méthyle, éthyle et trifluorométhyle, alkoxy tel que méthoxy ou éthoxy, un groupe acétylamine, un ou plusieurs atomes d'halogène tel que fluor, chlore, brome et iode. Aussi Y désigne un reste de formules $—OOC—R_6$ et $—O—R_6$, dans lesquelles $R_6$ peut désigner un reste acylamino tel qu'acétylamino.

Les sels des composés de formule (I) sont tous des sels d'addition d'acides non toxiques acceptables du point de vue physiologique. A titre d'exemples d'acides minéraux et organiques qui forment des sels avec les composés de formule (I) on mentionne les acides halogénhydrides tels que les acides chlorhydrique et bromhydrique, les acides phosphoniques, l'acide sulfurique, l'acide nitrique, les mono- et diacides carboxyliques et hydrocarboxyliques tels que l'acide acétique, l'acide maléique, l'acide succinique, l'acide fumarique, l'acide tartrique, l'acide citrique, l'acide salicylique, l'acide sorbique l'acide lactique, l'acide 1,5-naphtalènecarboxylique, l'acide méthanesulfonique et l'acide p-toluènesulfonique.

Les conditions de réaction employées dans les variantes a) à e), sont les suivantes:

On considère comme diluants l'eau et tous les solvants organiques inertes. Ce sont de préférence des alcools, par exemple le méthanol, l'éthanol, l'isopropanol et l'n-butanol; des éthers, par exemple des éthers dialkyliques inférieurs tels que l'éther diéthylique ou des éthers cycliques tels que le tétrahydrofurane et le dioxane, des acides carboxyliques aliphatiques inférieurs tels que les acides acétique et propionique, des dialkylformamides inférieurs telle que diméthylformamide, des alkylnitriles inférieurs tel que l'acétonitrile, le diméthylsulfoxyde, des bases hétéro-aromatiques liquides telle que la pyridine ainsi que des mélanges de ces solvants, y compris l'eau.

Dans le cas échéant, on peut faire réagir les réactifs sans diluant.

Les températures de réaction peuvent varier entre environ 20°C et environ 150°C, de préférence entre 50°C et 100°C, notamment à la température d'ébullition du solvant utilisé. La réaction peut être conduite à la pression normale, mais aussi sous pression élevée. On opère généralement à la pression normale. Les partenaires réactionnels sont utilisés de préférence en quantités à peu près molaires. L'ammoniac utilisé est ajouté avantageusement en excès de 1 à 2 moles. Les rapports molaires peuvent varier entre larges limites, sans nuire au résultat.

Le temps de réaction oscille entre 45 minutes et 10 heures.

Selon l'invention, on sépare et on isole le produit formé au cours de la réaction par des techniques qu'on utilise couramment dans ce but et on peut soumettre le produit à une purification classique, par exemple une recristallisation dans un solvant approprié ou dans un mélange de tels solvants.

La caractérisation des structures des différents composés (I) est réalisée par moyen des analyses élémentaires quantitatives et par les spectres I.R. et de R.M.N.

A titre de nouvelles substances actives, on mentionne, en plus des composés indiqués dans les exemples, les composés suivants:

2,6-diméthyl-5-méthoxycarbonyl-4-(3-pyridyle)-1,4-dihydropyridine-3-carboxylate de 2-(4-acétylaminophénoxy)éthyle,

2,6-diméthyl-5-éthoxycarbonyl-4-(3-trifluorométhylphényl)-1,4-dihydropyridine-3-carboxylate de 2-(4-acétylaminophénoxy)éthyle,

4-(2,3-dichlorophényl)-2,6-diéthyl-5-(2-tétrahydrofurfuryloxycarbonyl)-1,4-dihydropyridine-3-carboxylate de 2-(4-acétylaminophénoxy)éthyle,

2,6-diméthyl-4-(2-nitrophényl)-1,4-dihydropyridine-3,5-dicarboxylate de bis-2-(N-nicotynoylamino)-éthyle,

2,6-diméthyl-5-méthoxycarbonyl-4-(3-pyridyl)-1,4-dihydropyridine-3-carboxylate de 2-(N-nicotinoylamino)éthyle,

2,6-diméthyl-5-(2-méthoxyéthoxycarbonyl)-4-(3-trifluorométhylphényl)-1,4-dihydropyridine-3-carboxylate de 2-(N-nicotinoylamino)éthyle,

4-(2,3-dichlorophényl)-2,6-diméthyl-5-(2-méthylthioéthoxycarbonyl)-1,4-dihydropyridine-3-carboxylate de 2-(N-nicotinoylamino)éthyle,

2,6-diméthyl-5-méthoxycarbonyl-4-(3-pyridyl)-1,4-dihydropyridine-3-carboxylate de 2-(N-salicylamido)éthyle,

4-(2,3-dichlorophényl)-2,6-diméthyl-5-éthoxycarbonyl-1,4-dihydropyridine-3-carboxylate de 2-(N-salicylamido)éthyle,

2,6-diméthyl-5-méthoxycarbonyl-4-(2-pyridyl)-1,4-dihydropyridine-3-carboxylate de 2-[4-(2-furoyl)-1-pipérazinyl]éthyle,

2,6-diméthyl-5-éthoxycarbonyl-4-(3-trifluorométhylphényl)-1,4-dihydropyridine-3-carboxylate de 2-[4-(2-furoyl)-1-pipérazinyl]éthyle,

4-(2,3-dichlorophényl)-2,6-diméthyl-5-isopropoxycarbonyl-1,4-dihydropyridine-3-carboxylate de 2-[4-(2-furoyl)-1-pipérazinyl]éthyle,

4-(2,3-dichlorophényl)-2,6-diméthyl-1-[2-(N-morpholine)éthyl]-5-(2-méthoxyéthoxycarbonyl)-3-carboxylate de 2-(N-salicylamido)éthyle,

2,6-diméthyl-5-éthoxycarbonyl-4-(3-trifluorométhylphényl)-1,4-dihydropyridine-3-carboxylate de 2-(4-cinnamoyl-1-pipérarinyl)éthyle,

2,6-diméthyl-5-méthoxycarbonyl-4-(3-pyridyl)-1,4-dihydropyridine-3-carboxylate de 2-[4-(4-méthoxy)-cinnamoyl-1-pipérazinyl]éthyle,

4-(2,3-dichlorophényl)-2,6-diméthyl-5-(2-méthylthioéthoxycarbonyl)-1,4-dihydropyridine-3-carboxylate de 2-(4-cinnamoyl-1-pipérazinyl)éthyle.

Comme indiqué précédemment, i.e. a été découvert que les composés (I) pouvaient être avantageusement utilisés comme ingrédients pharmàcologiquement actifs dans des médicaments utilisés pour le traitement d'affections diverses. A titre d'exemple, on peut citer les affections vasculaires, notamment celles des vaisseaux coronaires sur lesquelles les composés (I) se sont montrés particulièrement actifs. De façon plus générale, la demanderesse a observé que les composés I ont des propriétés anti-athéromatiques et qu'ils peuvent exercer de ce fait un effet protecteur de nécroses.

Lors de tests sur animaux, il a été observé, que pour des doses thérapeutiques comparables, les composés I étaient plus actifs mais moins toxiques que des agents thérapeutiques similaires connus.

Pour obtenir les effets thérapeutiques souhaités, on peut administrer les composés I par voie orale par exemple, sous une forme appropriée. Les doses utilisées à cet effet peuvent contenir de 5 à 500 mg environ de produit actif par dose, selon les cas, en combinaison avec un support ou un excipient inerte, le cas échéant en combinaison avec un ingrédient pharmacologiquement actif additionnel.

De telles doses peuvent être utilisées soit pour un traitement préventif, soit pour un traitement curatif.

Exemple 1

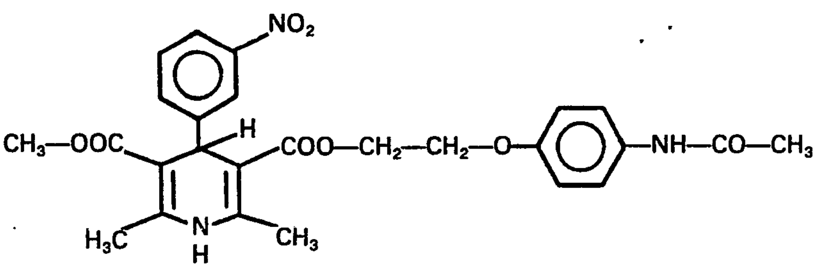

On chauffe à reflux pendant 4 heures une solution de 15 g (0.04 mol) de 2-(3-nitrobenzylidène)acétylacétate de 2-(4-acétylaminophénoxy)-éthyle et 4,19 g (0,04 mol) de 3-aminocrotonate de méthyle dans 40 ml d'éthanol. Après le refroidissement du mélange de réaction à 7°C, on obtient le 2,6-diméthyl-5-méthoxycarbonyl-4-(3-nitrophényl)-1,4-dihydropyridine-3-carboxylate de 2-(4-acétylaminophénoxy)éthyle, sous la forme de cristaux jaunes fondant à 202—204°C (recristallisè dans isopropanol); le rendement est de 68% de la théorie.

| Analyse pour $C_{26}H_{27}N_3O_8$: | %C | %H | %N |
|---|---|---|---|
| Calculé | 61,29 | 5,34 | 8,25 |
| Trouvé | 60,93 | 5,52 | 8,23 |

Spectre I.R. (KBr) ν($cm^{-1}$): 3360, 3210, 3080, 1700, 1670, 1510, 1350, 1240, 1210, 1090, 840, 780, 700.

Spectre R.M.N. (δ, $CDCl_3$ + DMSO-$d_6$) p.p.m.: 9,4 (1H, s); 8,6 (1H, s); 8 à 6,6 (8H, m); 5 (1H, s); 4,4 à 4 (4H, m); 3,6 (3H, s); 2,3 (6H, s); 2 (3H, s).

Exemple 2

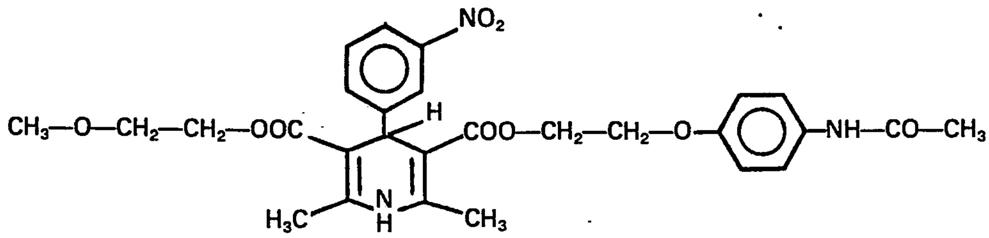

On chauffe à reflux pendant 8 heures une solution de 8,3 g (0,02 mol) de 2-(3-nitrobenzylidène)acétylacétate de 2-(4-acétylaminophénoxy)éthyle et 3,2 g (0,02 mol) de 3-aminocrotonate de 2-méthoxyéthyle dans 25 ml d'éthanol. Après le refroidissement du mélange de réaction à −5°C, on obtient de 2,6-diméthyl-5-(2-méthoxyéthoxycarbonyl)-4-(3-nitrophényl)-1,4-dihydropyridine-3-carboxylate de 2-(4-acétylamino-phénoxy)éthyle, sous la forme de poudre jaune fondant à 95—98°C (recristallisé dans éthanol); le rendement est de 64% de la théorie.

| Analyse pour $C_{28}H_{31}N_3O_9$: | %C | %H | %N |
|---|---|---|---|
| Calculé | 60,75 | 5,64 | 7,59 |
| Trouvé | 60,55 | 5,79 | 7,42 |

Spectre I.R. (KBr) ν($cm^{-1}$): 3170, 1710, 1700, 1540, 1520, 1360, 1220, 1120, 1100, 830, 710.

Spectre R.M.N. (δ, $CDCl_3$) p.p.m.: 8,2 à 6,6 (10H, m); 5,2 (1H, s); 4,4 à 4 (6H, m); 3,6 à 3,4 (2H, m); 3,3 (3H, s); 2,3 (6H, s); 2,1 (3H, s).

Exemple 3

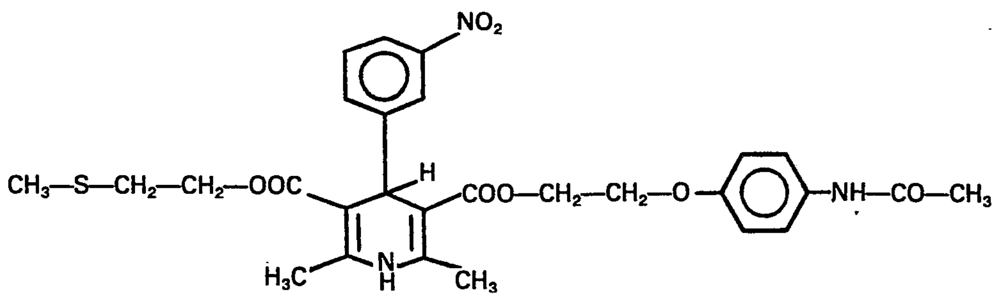

On chauffe à reflux pendant 8 heures une solution de 15 g (0,04 mol) de 2-(3-nitrobenzylidène)acétylacétate de 2-(4-acétylaminophénoxy)éthyle et 6,37 g (0,04 mol) de 3-aminocrotonate de 2-méthylthio éthyle dans 30 ml d'éthanol. Après le refrodissement du mélange de réaction à −5°C, on obtient le 2,6-diméthyl-5-(2-méthylthioéthoxycarbonyl)-4-(3-nitrophényl)-1,4-dihydropyridine-3-carboxylate de 2-(4-acétylamino-phénoxy)éthyle, sous la forme de cristaux jaunes fondant à 76—80°C (recristallisé d'éthanol); le rendement est de 80% de la théorie.

| Analyse pour $C_{26}H_{31}N_3O_8S$: | %C | %H | %N | %S |
|---|---|---|---|---|
| Calculé | 59,04 | 5,49 | 7,38 | 5,63 |
| Trouvé | 59,92 | 5,41 | 7,55 | 5,81 |

Spectre I.R. (KBr) ν($cm^{-1}$): 3320, 3100, 1700, 1670, 1530, 1510, 1350, 1210, 1120, 1010, 820, 700.

Spectre R.M.N. (δ, $CDCl_3$) p.p.m.: 8,1 à 6,6 (10H, m); 5,1 (1H, s); 4,4 à 4 (6H, m); 2,6 (2H, t); 2,3 (6H, s); 2,2 (3H, s); 2,1 (3H, s).

Exemple 4

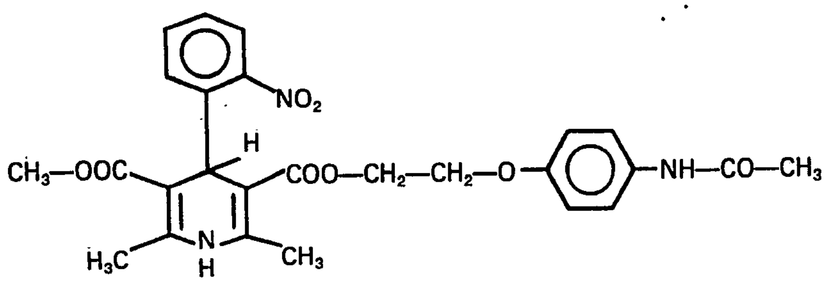

On chauffe à reflux pendant 10 heures une solution de 15 g (0,04 mol) de 2-(2-nitrobenzylidène)acétylacétate de 2-(4-acétylaminophénoxy)éthyle et 4,19 g (0,04 mol) de 3-aminocrotonate de méthyle dans 35 ml d'éthanol. Après le refroidissement du mélange de réaction à −5°C, on obtient le 2,6-diméthyl-5-mèthoxycarbonyl-4-(2-nitrophényl)-1,4-dihydropyridine-3-carboxylate de 2-(4-acétylaminophénoxy)éthyle, sous la forme de cristaux jaunes fondant à 163—166°C (recristallisé dans éthanol); le rendement est de 56% de la théorie.

| Analyse pour $C_{26}H_{27}N_3O_8$: | %C | %H | %N |
|---|---|---|---|
| Calculé | 61,29 | 5,34 | 8,25 |
| Trouvé | 61,27 | 5,36 | 7,94 |

Spectre I.R. (KBr) ν($cm^{-1}$): 3300, 3200, 1680, 1540, 1520, 1220, 1130, 1030, 840, 720.

Spectre R.M.N. (δ, $CDCl_3$ + DMSO-$d_6$) p.p.m.: 9,2 (1H, s); 8,2 (1H, s); 7,6 à 6,6 (8H, m); 5,7 (1H, s); 4,2 (4H, m); 3,5 (3H, s); 2,3 (6H, d); 2,1 (3H, s).

Exemple 5

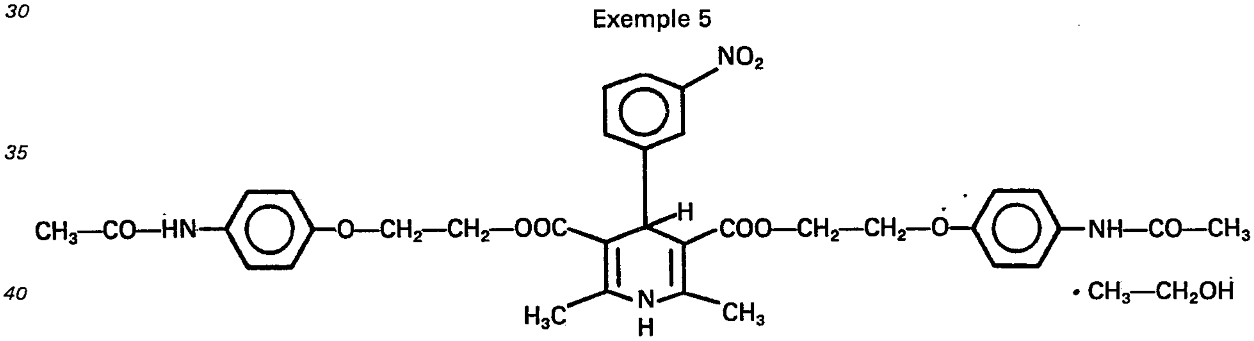

On chauffe à reflux pendant 1,5 heures une solution de 10 g (0,04 mol) de 3-aminocrotonate de 2-(4-acétylaminophénoxy)éthyle, 10,04 g (0,04 mol) d'acétylacétate de 2-(4-acétylaminophénoxy)éthyle et 5,43 g (0,04 mol) de 3-nitrobenzaldéhyde dans 35 ml d'éthanol. Après le refroidissement à la température ambiante, on obtient le 2,6-diméthyl-4-(3-nitrophényl)-1,4-dihydropyridine-3,5-dicarboxylate de bis-2-(4-acétylaminophénoxy)éthyle avec une molécule d'éthanol, sous la forme de poudre jaune pâle fondant à 142—152°C (recristallisé dans éthanol), le rendement est de 50% de la théorie.

| Analyse pour $C_{35}H_{36}N_4O_{10}.C_2H_6O$: | %C | %H | %N |
|---|---|---|---|
| Calculé | 61,83 | 5,89 | 7,79 |
| Trouvé | 62,08 | 5,79 | 8,02 |

Spectre I.R. (KBr) ν($cm^{-1}$): 3330, 1690, 1670, 1540, 1520, 1350, 1250, 1120, 830, 720.

Spectre R.M.N. (δ, DMSO-$d_6$) p.p.m.: 9,7 (2H, s); 9 (1H, s); 8 à 6,6 (12H, m); 5 (1H, s); 4,4 à 4 (8H, m); 3,4 (2H, q); 2,3 (6H, s); 2 (6H, s); 1,1 (3H, t).

Exemple 6

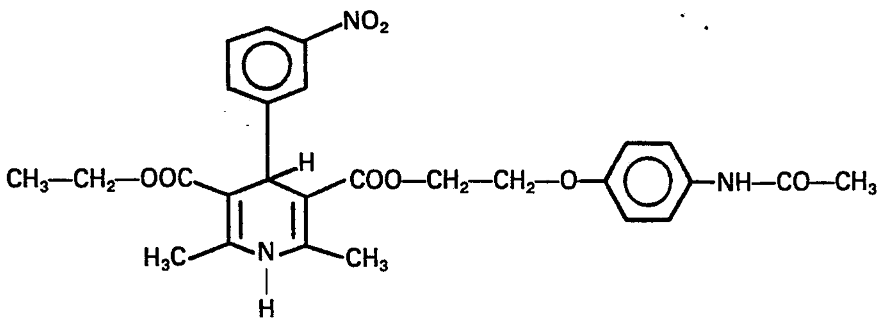

On chauffe à reflux pendant 8 heures une solution de 12,28 g (0,03 mol) de 2-(3-nitrobenzylidène)-acétylacétate de 2-(4-acétylaminophénoxy)éthyle et 3,85 g (0,03 mol) de 3-aminocrotonate d'éthyle dans 30 ml d'éthanol. Après le refroidissement du mélange de réaction a −5°C, on obtient le 2,6-diméthyl-5-éthoxy-carbonyl-4-(3-nitrophényl)-1,4-dihydropyridine-3-carboxylate de 2-(4-acétylaminophénoxy)éthyle, sous la forme de cristaux jaunes fondant à 198—200°C (recristallisé dans éthanol); le rendement est de 75% de la théorie.

| Analyse pour $C_{27}H_{29}N_3O_8$: | %C | %H | %N |
|---|---|---|---|
| Calculé | 61,94 | 5,58 | 8,03 |
| Trouvé | 61,88 | 5,87 | 7,55 |

Spectre I.R. (KBr) ν($cm^{-1}$): 3360, 3280, 3220, 3080, 1695, 1665, 1530, 1510, 1350, 1240, 1200, 1090, 930, 840, 780, 700.

Spectre R.M.N. (δ, $CDCl_3$) p.p.m.: 9,7 (1H, s); 9 (1H, s); 8 à 6,6 (8H, m); 5 (1H, s); 4,2 (6H, m); 2,4 (6H, s); 2,1 (3H, s); 1,1 (3H, t).

Exemple 7

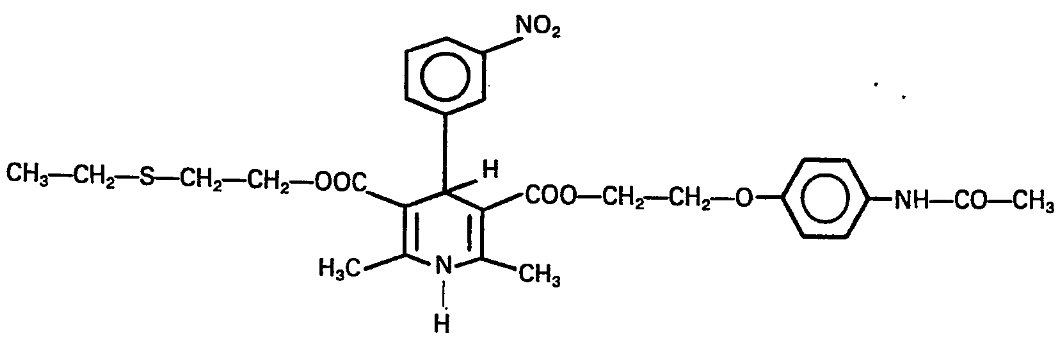

On chauffe à reflux pendant 8 heures une solution de 15 g (0,04 mol) de 2-(3-nitrobenzylidène)acétylacétate de 2-(4-acétylaminophénoxy)éthyle et 6,88 g (0,04 mol) de 3-aminocrotonate de 2-éthylthioéthyle dans 35 ml d'éthanol. Après le refroidissement du mélange de réaction à −5°C, on obtient le 2,6-diméthyl-5-(2-éthylthioéthoxycarbonyl)-4-(3-nitrophényl)-1,4-dihydropyridine-3-carboxylate de 2-(4-acétylamino-phénoxy)éthyle, sous la forme de poudre jaune fondant à 100—104°C (recristallisé dans éthanol); le rendement est de 60% de la théorie.

| Analyse pour $C_{29}H_{33}N_3O_8S$: | %C | %H | %N | %S |
|---|---|---|---|---|
| Calculé | 59,68 | 5,70 | 7,20 | 5,49 |
| Trouvé | 59,19 | 6,02 | 7,19 | 5,90 |

Spectre I.R. (KBr) ν($cm^{-1}$): 3440, 3340, 3150, 1730, 1700, 1560, 1380, 1250, 1150, 860, 740.

Spectre R.M.N. (δ, $CDCl_3$) p.p.m.: 8 à 6,6 (10H, m); 5,1 (1H, s); 4,4 à 4 (6H, m); 2,6 (4H, m); 2,3 (6H, s); 2,1 (3H, s); 1,2 (3H, t).

Exemple 8

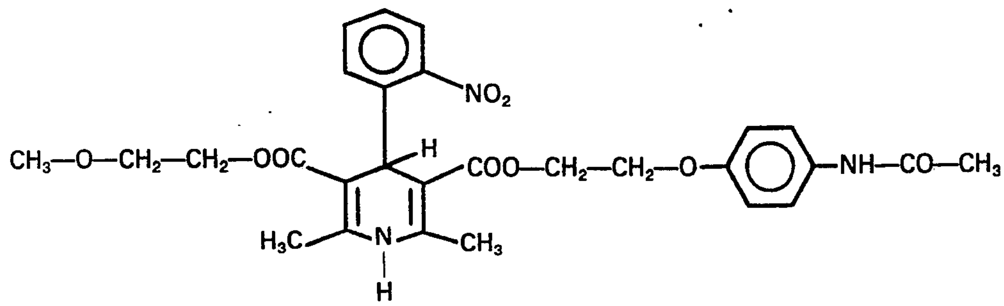

On chauffe à reflux pendant 10 heures une solution de 15 g (0,04 mol) de 2-(2-nitrobenzylidène)acétylacétate de 2-(4-acétylaminophénoxy)éthyle et 5,79 g (0,04 mol) de 3-aminocrotonate de 2-méthoxyéthyle dans 35 ml d'éthanol. Après le refroidissement à la température ambiante, on obtient le 2,6-diméthyl-5-(2-méthoxyéthoxycarbonyl)-4-(2-nitrophényl)-1,4-dihydropyridine-3-carboxylate de 2-(4-acétylaminophénoxy)-éthyle, sous la forme de cristaux jaunes fondant à 94—105°C avec décomposition (recristallisé dans éthanol); le rendement est de 84% de la théorie.

| Analyse pour $C_{28}H_{31}N_3O_9$: | %C | %H | %N |
|---|---|---|---|
| Calculé | 60,75 | 5,64 | 7,59 |
| Trouvé | 60,48 | 5,64 | 7,59 |

Spectre I.R. (KBr) ν($cm^{-1}$): 3300, 1710, 1665, 1540, 1520, 1250, 1200, 890, 830, 720.

Spectre R.M.N. (δ, DMSO-$d_6$) p.p.m.: 9,8 (1H, s); 9 (1H, s); 7,6 à 6,6 (8H, m); 5,6 (1H, s); 4,1 (6H, m); 3,4 (2H, m); 3,1 (3H, s); 2,2 (6H, s); 2 (3H, s).

Exemple 9

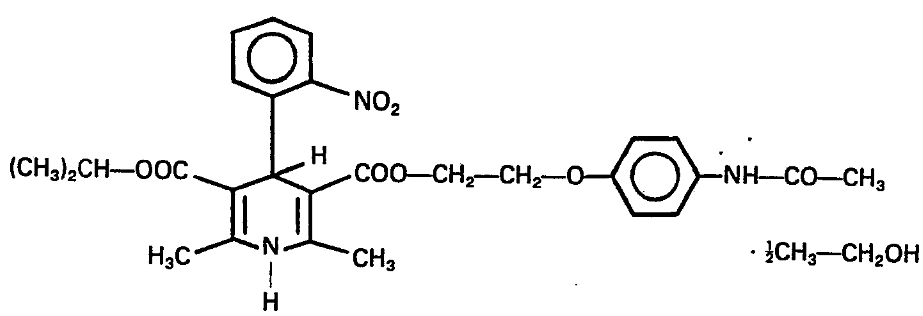

$\cdot \frac{1}{2} CH_3—CH_2OH$

On chauffe à reflux pendant 10 heures une solution de 15 g (0,04 mol) de 2-(2-nitrobenzylidène)acétylacétate de 2-(4-acétylaminophénoxy)éthyle et 5,21 g (0,04 mol) de 3-aminocrotonate d'isopropyle dans 35 ml d'éthanol. Après le refroidissement à −5°C, on obtient le 2,6-diméthyl-5-isopropoxycarbonyl-4-(2-nitrophènyl)-1,4-dihydropyridine-3-carboxylate de 2-(4-acétylaminophénoxy)éthyle avec une demie molécule d'éthanol, sous la forme de cristaux jaunes fondant à 103—112°C (recristallisé dans éthanol); le rendement est de 35% de la théorie.

| Analyse pour $C_{28}H_{31}N_3O_8 . \frac{1}{2} C_2H_6O$: | %C | %H | %N |
|---|---|---|---|
| Calculé | 62,13 | 6,11 | 7,50 |
| Trouvé | 62,05 | 5,98 | 7,82 |

Spectre I.R. (KBr) ν($cm^{-1}$): 3350, 1705, 1540, 1520, 1210, 1110, 835, 720.

Spectre R.M.N. (δ, $CDCl_3$) p.p.m.: 7,9 à 6,4 (10H, m); 5,8 (1H, s); 4,9 (1H, h); 4,3 (2H, sl); 4,1 (2H, sl); 3,7 ($\frac{1}{2}$ 2H, q); 2,3 (6H, d); 2,1 (3H, s); 1,3 à 0,9 (6H + $\frac{1}{2}$ 3H, d + t).

Exemple 10

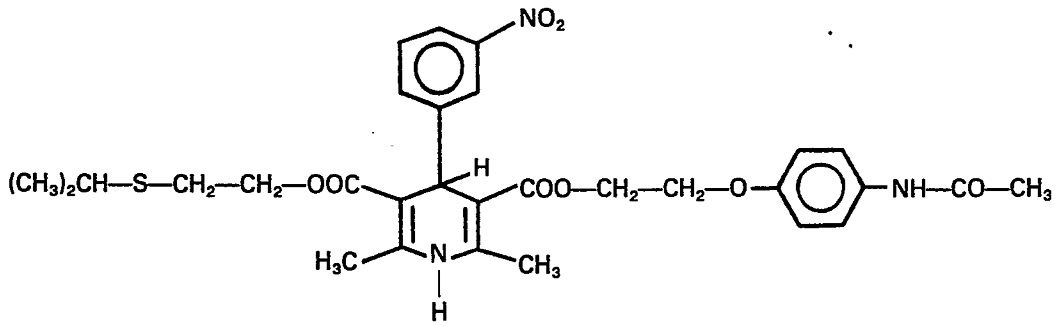

On chauffe à reflux pendant 8 heures une solution de 12 g (0,03 mol) de 2-(3-nitrobenzylidène)acétylacétate de 2-(4-acétylaminophénoxy)éthyle et 5,92 g (0,03 mol) de 3-aminocrotonate de 2-isopropylthioéthyle dans 30 ml d'éthanol. Après le refroidissement à −5°C, on obtient le 2,6-diméthyl-5-(2-isopropylthioéthoxycarbonyl)-4-(3-nitrophényl)-1,4-dihydropyridine-3-carboxylate de 2-(4-acétylaminophénoxy)éthyle, sous la forme de cristaux jaunes fondant à 142—144°C (recristallisé dans éthanol aqueux). Le rendement est de 42% de la théorie.

| Analyse pour $C_{30}H_{35}N_3O_8S$: | %C | %H | %N | %S |
|---|---|---|---|---|
| Calculé | 60,29 | 5,90 | 7,03 | 5,36 |
| Trouvé | 60,35 | 6,10 | 6,97 | 5,65 |

Spectre I.R. (KBr) ν($cm^{-1}$): 3300, 3260, 3100, 1670, 1620, 1530, 1510, 1350, 1210, 1120, 1020, 920, 820, 750, 700.

Spectre R.M.N. (δ, $CDCl_3$ + DMSO-$d_6$) p.p.m.: 9,2 (1H, s); 8,3 (1H, s); 8 à 6,6 (8H, m); 5,1 (1H, s); 4,4 à 4 (6H, m); 3 à 2,5 (3H, m); 2,35 (6H, s); 2,1 (3H, s); 1,2 (6H, d).

Exemple 11

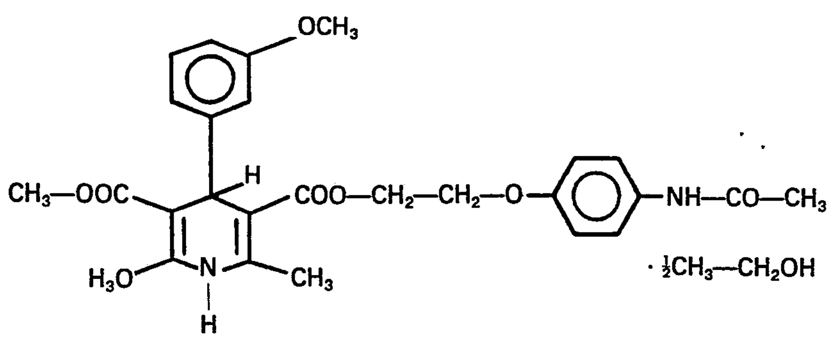

On chauffe à reflux pendant 8 heures une solution de 10 g (0,04 mol) de 2-(3-méthoxybenzylidène)-acétylacétate de méthyle et 11,88 g (0,04 mol) de 3-aminocrotonate de 2-(4-acétylaminophénoxy)éthyle dans 50 ml d'éthanol. Après le refroidissement à −5°C, on obtient le 2,6-diméthyl-5-méthoxycarbonyl-4-(3-méthoxyphényl)-1,4-dihydropyridine-3-carboxylate de 2-(4-acétylaminophénoxy)éthyle, avec une demie molécule d'éthanol, sous la forme d'aiguilles blanches fondant à 88—92°C (recristallisé dans éthanol); le rendement est de 50% de la théorie.

| Analyse pour $C_{27}H_{30}N_2O_7 \cdot \frac{1}{2}C_2H_6O$: | %C | %H | %N |
|---|---|---|---|
| Calculé | 64,98 | 6,43 | 5,41 |
| Trouvé | 64,80 | 6,68 | 5,44 |

Spectre I.R. (KBr) ν($cm^{-1}$): 3400, 1710, 1670, 1520, 1500, 1220, 1120, 1055, 830, 780, 720.

Spectre R.M.N. (δ, DMSO-$d_6$) p.p.m.: 9,7 (1H, s); 8,8 (1H, s); 7,6 à 6,6 (8H, m); 4,9 (1H, s); 4,2 (4H, m); 3,65 (3H, s); 3,55 (3H, s); 2,3 (6H, s); 2 (3H, s); 1,1 ($\frac{1}{2}$3H, t).

Exemple 12

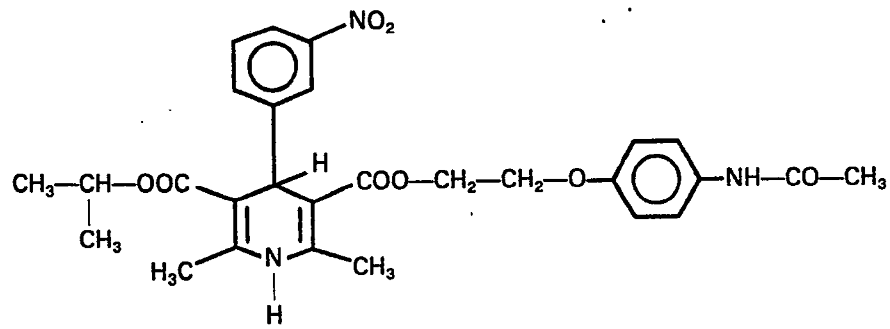

On chauffe à reflux pendant 8 heures une solution de 15 g (0,04 mol) de 2-(3-nitrobenzylidène)acétylacétate de 2-(4-acétylaminophénoxy)éthyle et 5,21 g (0,04 mol) de 3-aminocrotonate d'isopropyle dans 35 ml d'éthanol. Après l'évaporation de 15 ml du solvant employé et refroidissement à −5°C, on obtient le 2,6-diméthyl-5-isopropoxycarbonyl-4-(3-nitrophényl)-1,4-dihydropyridine-3-carboxylate de 2-(4-acétylaminophénoxy)éthyle sous forme de poudre jaune pâle fondant à 146—148°C (recristallisé dans éthanol); le rendement est de 50% de la théorie.

| Analyse pour $C_{28}H_{31}N_3O_8$: | %C | %H | %N |
|---|---|---|---|
| Calculé | 62,56 | 5,81 | 7,82 |
| Trouvé | 62,89 | 5,99 | 8,03 |

Spectre I.R. (KBr) ν(cm$^{-1}$): 3400, 3300, 3080, 1700, 1675, 1530, 1510, 1350, 1240, 1210, 1100, 1070, 840, 790, 700.

Spectre R.M.N. (δ, $CDCl_3$) p.p.m.: 8 à 6,5 (10H, m); 5 (1H + 1H, s + m); 4,3 (2H, m); 4,05 (2H, m); 2,3 (6H, s); 2,1 (3H, s); 1,1 (6H, q).

Exemple 13

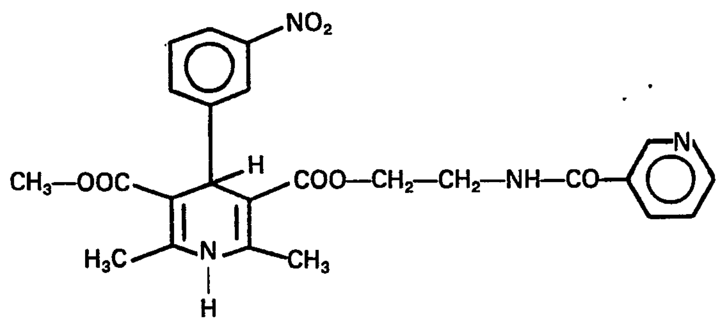

On chauffe à reflux pendant 45 minutes une solution de 25 g (0,07 mol) de 2-(3-nitrobenzylidène)acétylacétate de 2-(N nicotinoylamino)éthyle et 7,51 g (0,07 mol) de 3-aminocrotonate de méthyle dans 70 ml d'éthanol. Après le refroidissement à la température ambiante, on obtient le 2,6-diméthyl-5-méthoxycarbonyl-4-(3-nitrophényl)-1,4-dihydropyridine-3-carboxylate de 2-(N-nicotinoylamino)éthyle, sous la forme de cristaux jaunes fondant à 207—209°C (recristallisé dans éthanol); le rendement est de 70% de la théorie.

| Analyse pour $C_{24}H_{24}N_4O_7$: | %C | %H | %N |
|---|---|---|---|
| Calculé | 60,00 | 5,03 | 11,66 |
| Trouvé | 59,99 | 5,20 | 11,20 |

Spectre I.R. (KBr) ν(cm$^{-1}$): 3440, 3220, 3080, 2960, 1700, 1670, 1510, 1350, 1210, 1120, 1040, 780, 750, 700.

Spectre R.M.N. (δ, DMSO-$d_6$) p.p.m.: 9,2 à 7,3 (10H, m); 5,1 (1H, s); 4,2 (2H, m); 3,6 (5H, s); 2,4 (6H, s).

Exemple 14

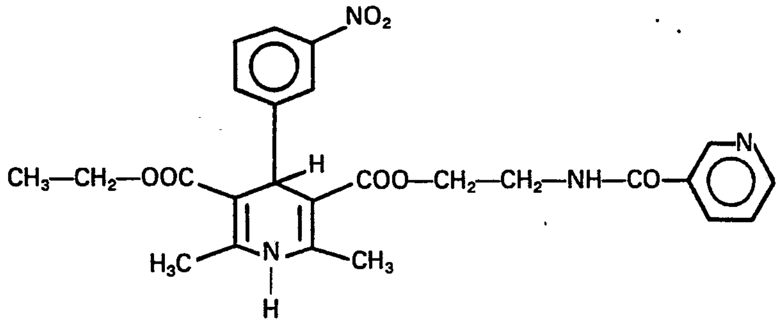

On chauffe à reflux pendant 8 heures une solution de 10 g (0,03 mol) de 2-(3-nitrobenzylidène)acétylacétate de 2-(N-nicotinoylamino)éthyle et 3,37 g (0,03 mol) de 3-aminocrotonate d'éthyle dans 35 ml d'éthanol. Après l'évaporation de 15 ml du solvant et refroidissement à −5°C, on obtient le 2,6-diméthyl-5-éthoxycarbonyl-4-(3-nitrophényl)-1,4-dihydropyridine-3-carboxylate de 2-(N-nicotinoylamino)éthyle, sous la forme de cristaux jaunes fondant à 153—155°C (recristallisé dans éthanol); le rendement est de 32% de la théorie.

| Analyse pour $C_{25}H_{26}N_4O_7$: | %C | %H | %N |
|---|---|---|---|
| Calculé | 60,72 | 5,30 | 11,33 |
| Trouvé | 61,02 | 5,46 | 11,53 |

Spectre I.R. (KBr) v($cm^{-1}$): 3300, 3220, 3080, 1690, 1640, 1530, 1490, 1145, 1200, 1090, 780, 740, 700.

Spectre R.M.N. (δ, $CDCl_3$ + DMSO-$d_6$) p.p.m.: 9 à 7,2 (10H, m); 5,1 (1H, s); 4,3 à 3,4 (6H, m); 2,35 (6H, s); 1,2 (3H, t).

Exemple 15

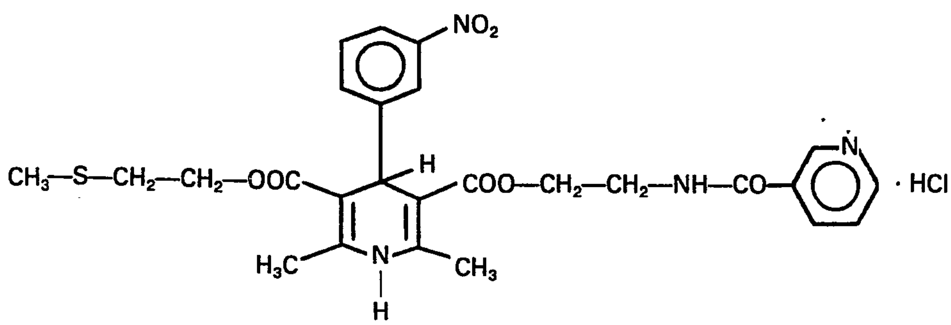

On chauffe à reflux pendant 10 heures une solution de 10,79 g (0,03 mol) de 2-(3-nitrobenzylidène)-acétylacétate de 2-(N-nicotinoylamino)éthyle et 4,93 g (0,03 mol) de 3-aminocrotonate de 2-méthylthio-éthyle dans 30 ml d'éthanol. Après l'évaporation du solvant résulte une huile orange qui se transforme en son chlorhydrate par dissolution d'abord dans 100 ml d'éthanol absolu et puis addition de 100 ml d'éther éthylique saturés de chlorure d'hydrogène. L'évaporation du solvant donne un produit qui se recristallise d'éthanol-éther et on obtient ainsi le chlorhydrate du 2,6-diméthyl-5-(2-mèthylthioéthoxycarbonyl)-4-(3-nitrophényl)-1,4-dihydropyridine-3-carboxylate de 2-(N-nicotinoylamino)éthyle sous la forme de cristaux jaunes fondant à 120—130°C avec décomposition; le rendement est de 25% de la théorie.

| Analyse pour $C_{26}H_{28}N_4O_7S.HCl$: | %C | %H | %N | %S | %Cl |
|---|---|---|---|---|---|
| Calculé | 54,12 | 5,07 | 9,71 | 5,56 | 6,14 |
| Trouvé | 54,33 | 5,18 | 9,89 | 4,65 | 6,24 |

Spectre I.R. (KBr) v($cm^{-1}$): 3380, 3250, 3080, 1685, 1539, 1490, 1350, 1220, 1120, 1020, 830, 740, 710, 680.

Spectre R.M.N. (δ, $CDCl_3$ + DMSO-$d_6$) p.p.m.: 13,6 (1H, sl); 9,4 à 7,2 (10, m); 5,05 (1H, s); 4,2 (4H, m); 3,6 (2H, m); 2,6 (2H, t); 2,4 (6H, s); 2,1 (3H, s).

Exemple 16

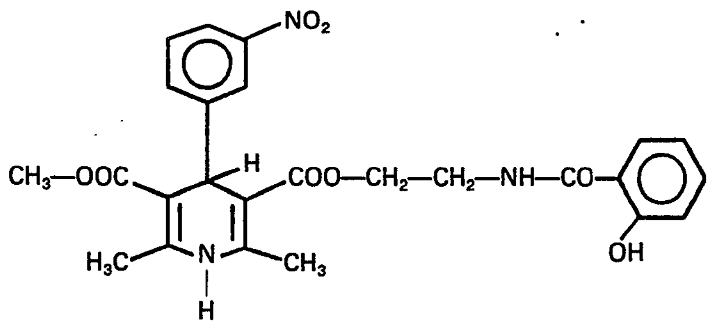

On chauffe à reflux pendant 6 heures une solution de 14,15 g (0,06 mol) de 2-(3-nitrobenzylidène)-acétylacétate de méthyle et 15 g (0,06 mol) de 3-aminocrotonate de 2-(N-salicylamido)éthyle, dans 55 ml d'éthanol. Après l'évaporation du solvant, l'huile résultant est dissoute dans 10 ml de méthanol à ébullition et refroidit à la température ambiante, pour obtenir ainsi le 2,6-diméthyl-5-méthoxycarbonyl-4-(3-nitrophényl)-1,4-dihydropyridine-3-carboxylate de 2-(N-salicylamido)éthyle sous la forme de cristaux jaunes fondant à 165—170°C (recristallisé dans éthanol); le rendement est de 83% de la théorie.

| Analyse pour $C_{25}H_{25}N_3O_8$: | %C | %H | %N |
|---|---|---|---|
| Calculé | 60,60 | 5,09 | 8,48 |
| Trouvé | 60,54 | 5,12 | 8,70 |

Spectre I.R. (KBr) ν($cm^{-1}$): 3460, 3380, 1710, 1660, 1545, 1490, 1360, 1210, 1130, 1095, 755, 705.

Spectre R.M.N. (δ, $CDCl_3$) p.p.m.: 12,3 (1H, s); 8,6 à 6,7 (10H, m); 5,1 (1H, s); 4,2 (2H, m); 3,55 (5H, sd); 2,35 (6H, s).

Exemple 17

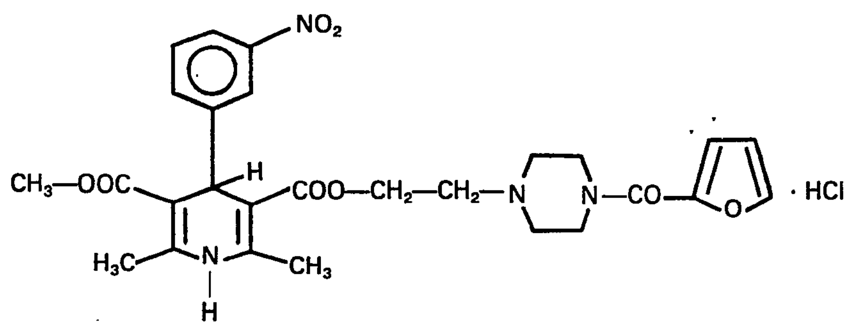

On chauffe à reflux pendant 8 heures une solution de 12 g (0,05 mol) de 2-(3-nitrobenzylidène)acétylacétate de méthyle et 14, 8 g (0,05 mol) de 3-aminocrotonate de 2-[4-(2-furoyl)-1-pipérazinyl]éthyle dans 45 ml d'éthanol. Après l'évaporation du solvant employé, l'huile résultant se transforme en son chlorhydrate par dissolution d'abord dans 100 ml d'éther éthylique saturés de chlorure d'hydrogène; l'évaporation du solvant donne un produit qui se triture avec éthanol à ébullition pour obtenir ainsi le chlorhydrate du 2,6-diméthyl-5-méthoxycarbonyl-4-(3-nitrophényl)-1,4-dihydropyridine-3-carboxylate de 2-[4-(2-furoyl)-1-pipérazinyl)éthyle sous la forme de poudre jaune pâle fondant à 237—240°C avec décomposition; le rendement est de 40% de la théorie.

| Analyse pour $C_{27}H_{30}N_4O_8.HCl$: | %C | %H | %N | %Cl |
|---|---|---|---|---|
| Calculé | 56,40 | 5,43 | 9,74 | 6,17 |
| Trouvé | 56,71 | 5,60 | 10,42 | 6,41 |

Spectre I.R. (KBr) ν($cm^{-1}$): 3240, 3120, 2600, 2530, 1710, 1670, 1540, 1495, 1360, 1220, 1135, 890, 760, 710.

Spectre R.M.N. (δ, DMSO-$d_6$) p.p.m.: 9,2 (1H, sl); 8 à 6,5 (8H, m); 5 (1H, s); 4,4 (4H, sl); 3,7 à 3 (3H + 8H, m); 2,3 (6H, d).

Exemple 18

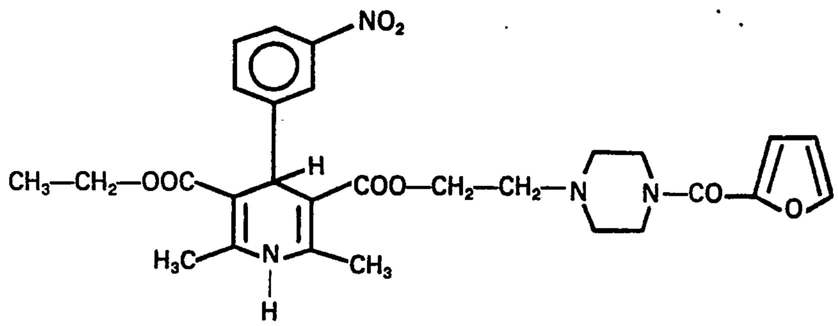

On chauffe à reflux pendant 7 heures une solution de 17,32 g (0,07 mol) de 2-(3-nitrobenzylidène)-acétylacétate d'éthyle et 20,22 g (0,07 mmol) de 3-aminocrotonate de 2-[4-(2-furoyl)-1-pipérazinyl]éthyle dans 60 ml d'éthanol. Après le refroidissement à −5°C, on obtient le 2,6-diméthyl-5-éthoxycarbonyl-4-(3-nitrophényl)-1,4-dihydropyridine-3-carboxylate de 2-[4-(2-furoyl)-1-pipérazinyl]éthyle sous forme de cristaux jaunes fondant à 149—152°C (recristallisé d'éthanol); le rendement est de 65% de la théorie.

| Analyse pour $C_{28}H_{32}N_4O_8$: | %C | %H | %N |
|---|---|---|---|
| Calculé | 60,86 | 5,84 | 10,14 |
| Trouvé | 61,14 | 5,97 | 10,08 |

Spectre I.R. (KBr) ν(cm$^{-1}$): 3280, 3220, 3100, 2980, 1700, 1620, 1540, 1500, 1360, 1280, 1210, 1110, 1030, 790, 750, 720.

Spectre R.M.N. (δ, $CDCl_3$) p.p.m.: 8,1 à 6,4 (8H, m); 5,1 (1Hs); 4,2 (4H, m); 3,7 (4H, m); 2,7 à 2,2 (6H + 6H, m + s); 1,2 (3H, t).

Exemple 19

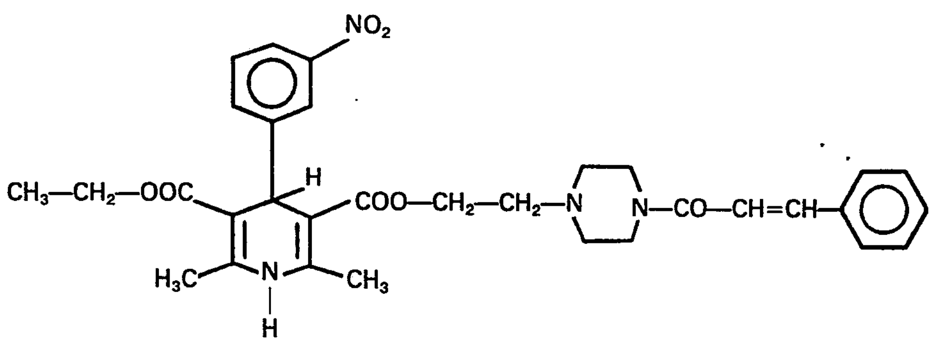

On chauffe à reflux pendant 8 heures, 11,78 g (0,04 mol) de 2-(3-nitrobenzylidène)acétylacétate d'éthyle et 15,37 g (0,04 mol) de 3-aminocrotonate de 2-(4-cinnamoyl-1-pipérazinyl)éthyle dans 45 ml d'éthanol. Après le refrodissement à −5°C, on obtient le 2,6-diméthyl-5-éthoxycarbonyl-4-(3-nitrophényl)-1,4-dihydropyridine-3-carboxylate de 2-(4-cinnamoyl-1-pipérazinyl)éthyle sous la forme de cristaux jaunes fondant à 164—172°C (recristallisé dans éthanol); le rendement est de 56% de la théorie.

| Analyse pour $C_{32}H_{36}N_4O_7$: | %C | %H | %N |
|---|---|---|---|
| Calculé | 65,29 | 6,16 | 9,52 |
| Trouvé | 65,49 | 6,20 | 9,78 |

Spectre I.R. (KBr) ν(cm$^{-1}$): 3300, 3250, 3120, 1710, 1650, 1600, 1540, 1360, 1280, 1210, 1100, 785, 760, 715.

Spectre R.M.N. (δ, $CDCl_3$) p.p.m.: 8,2 à 6,7 (12H, m); 5,1 (1H, s); 4,2 (4H, m); 3,6 (4H, s); 2,6 à 2,2 (6H + 6H, m + s); 1,2 (3H, t).

Exemple 20

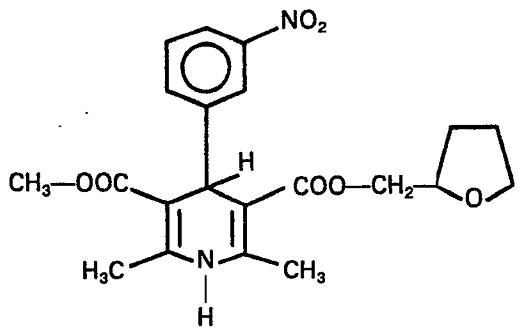

On chauffe à reflux pendant 8 heures une solution de 15 g (0,05 mol) de 2-(3-nitrobenzylidène)acétylacétate de 2-tétrahydrofurfuryle et 5,41 g (0,05 mol) de 3-aminocrotonate de méthyle dans 50 ml d'éthanol. Après le refroidissement du mélange de réaction à −5°C, on obtient le 2,6-diméthyl-5-méthoxycarbonyl-4-(3-nitrophényl)-1,4-dihydropyridine-3-carboxylate de 2-tétrahydrofurfuryle, sous la forme de cristaux jaunes fondant à 135—140°C (recristallisé dans éthanol); le rendement est de 59% de la théorie.

| Analyse pour $C_{21}H_{24}N_2O_7$: | %C | %H | %N |
|---|---|---|---|
| Calculé | 60,57 | 5,81 | 6,73 |
| Trouvé | 60,51 | 5,99 | 6,39 |

Spectre I.R. (KBr) ν($cm^{-1}$): 3470, 3100, 2950, 2870, 1710, 1650, 1530, 1480, 1345, 1220, 1120, 1020, 825, 780, 740, 700, 680.

Spectre R.M.N. (δ, $CDCl_3$) p.p.m.: 8,1 à 7,2 (4H, m); 6,8 (1H, s); 5,1 (1H, s); 4,1 (3H, sd); 3,9 à 3,6 (2H + 3H, m + s); 2,35 (6H, s); 1,8 (4H, m).

Exemple 21

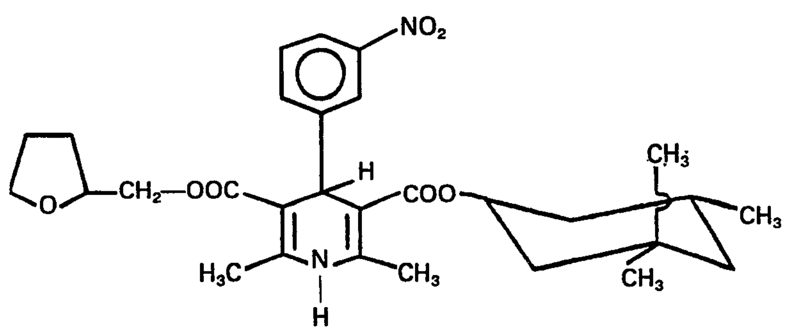

On chauffe à reflux pendant 12 heures une solution de 15 g (0,08 mol) de 3-aminocrotonate de 2-tétrahydrofurfuryle, 18,33 g (0,08 mol) d'acétylacétate de 3,3,5-triméthylcyclohéxyle et 12,24 g (0,08 mol) de 3-nitrobenzaldéhyde dans 65 ml d'éthanol. Après l'addition de 35 ml de $H_2O$ sur le mélange de réaction et refroidissement à −5°C, résulte une huile jaune qui se sépare par décantation et se sèche sous vide dans un dessicateur. On obtient ainsi le 2,6-diméthyl-4-(3-nitrophényl)-5-(2-tétrahydrofurfuryloxycarbonyl)-1,4-dihydropyridine-3-carboxylate de 3,3,5-triméthylcyclohéxyle, sous la forme de cristaux jaunes fondant à 159—162°C (recristallisé dans éthanol); le rendement est de 30% de la théorie.

| Analyse pour $C_{29}H_{38}N_2O_7$: | %C | %H | %N |
|---|---|---|---|
| Calculé | 66,14 | 7,27 | 5,32 |
| Trouvé | 66,41 | 7,57 | 5,23 |

Spectre I.R. (KBr) ν($cm^{-1}$): 3380, 2950, 1700, 1520, 1490, 1345, 1200, 1090, 790, 740, 710.

Spectre R.M.N. (δ, $CDCl_3$) p.p.m.: 8,1 à 7,2 (4H, m); 6,5 (1H, s); 5,2 (1H, d); 5 (1H, s); 4,1 à 3,8 (s + t, 3H + 2H); 2,3 (6H, d); 2 à 0,6 (m, 20H).

Exemple 22

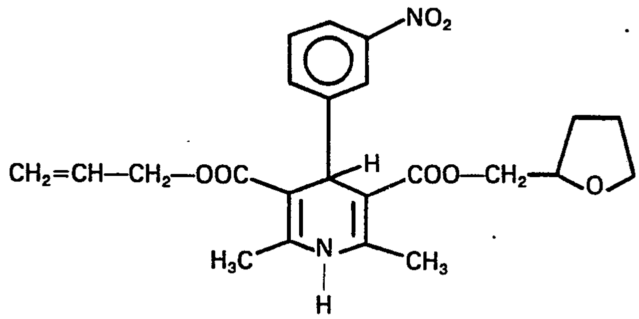

On chauffe à reflux pendant 12 heures une solution de 15 g (0,08 mol) de 3-aminocrotonate de 2-tétrahydrofurfuryle, 11,51 g (0;08 mol) de acétylacétate d'allyle et 12,24 g (0,08 mol) de 3-nitrobenzaldéhyde dans 65 ml d'éthanol. Après l'évaporation de 30 ml du solvant et refroidissement du mélange de réaction à −5°C, on obtient un produit qui est purifié par chromatographie à colonne sur silicagel-60 en benzène, (éluant:benzène/acétate d'éthyle (9:1)). On obtient ainsi le 5-allyloxycarbonyl-2,6-diméthyl-4-(3-nitrophényl)-1,4-dihydropyridine-3-carboxylate de 2-tétrahydrofurfuryle, sous la forme de cristaux jaunes fondant à 129—131°C; le rendement est de 26% de la théorie.

| Analyse pour $C_{23}H_{26}N_2O_7$: | %C | %H | %N |
|---|---|---|---|
| Calculé | 62,43 | 5,92 | 6,33 |
| Trouvé | 62,67 | 5,87 | 6,53 |

Spectre I.R. (KBr) $\nu$(cm$^{-1}$): 3340, 3260, 2950, 2880, 1700, 1660, 1530, 1350, 1210, 1090, 1020, 780, 750, 700.

Spectre R.M.N. ($\delta$, $CDCl_3$) p.p.m.: 8,1 à 7,2 (4H, m); 6,6 (1H, s); 5,7 (1H, m); 5,3 à 4,9 (4H, m); 4,5 (2H, d); 4,1 (2H, s); 3,8 (2H, td); 2,4 (6H, s); 1,9 (4H, m).

Exemple 23

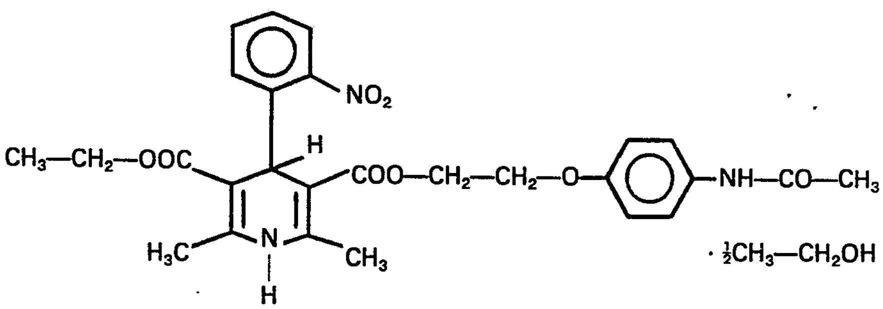

On chauffe à reflux pendant 8 heures une solution de 8,02 g (0,03 mol) de 2-(2-nitrobenzylidène)acétylacétate d'éthyle et 9,65 g (0,03 mol) de 3-aminocrotonate de 2-(4-acétylaminophénoxy)éthyle dans 40 ml d'éthanol. Aprés le refroidissement du mélange de réaction à −5°C, on obtient le 2,6-diméthyl-5-éthoxycarbonyl-4-(2-nitrophényl)-1,4-dihydropyridine-3-carboxylate de 2-(4-acétylaminophénoxy)éthyle, avec une demie molécule d'éthanol, sous la forme de cristaux jaunes fondant à 96—108°C (recristallisé dans éthanol); le rendement est de 57% de la théorie.

| Analyse pour $C_{27}H_{29}N_3O_8 . \frac{1}{2} C_2H_6O$: | %C | %H | %N |
|---|---|---|---|
| Calculé | 61,53 | 5,90 | 7,69 |
| Trouvé | 61,08 | 6,07 | 7,45 |

Spectre I.R. (KBr) $\nu$(cm$^{-1}$): 3380, 3000, 1700, 1540, 1520, 1320, 1220, 1120, 1025, 835, 760, 715.

Spectre R.M.N. ($\delta$, $CDCl_3$) p.p.m.: 8,2 à 6,5 (9H, m); 5,8 (1H, s); 4,4 à 3,8 (7H, m); 2,3 (6H, s); 2,1 (3H, s); 1,1 (4, 5H, t).

Exemple 24

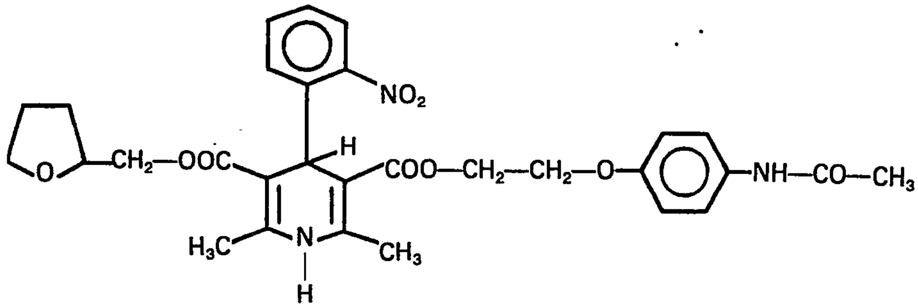

On chauffe à reflux pendant 8 heures une solution de 15 g (0,05 mol) de 2-(2-nitrobenzylidène)acétylacétate de 2-tétrahydrofurfuryle et 13, 07 g (0,05 mol) de 3-aminocrotonate de 2-(4-acétylaminophénoxy)-éthyle dans 50 ml d'éthanol. Après le refroidissement du mélange de réaction à la température ambiante, on obtient le 2,6-(diméthyl-4-(2-nitrophényl)-5-(2-tétrahydrofurfuryloxycarbonyl)-1,4-dihydropyridine-3-carboxylate de 2-(4-acétylaminophénoxy)éthyle, sous la forme d'aiguilles jaunes fondant à 146—150°C (recristallisé dans éthanol); le rendement est de 57% de la théorie.

| Analyse pour $C_{30}H_{33}N_3O_9$: | %C | %H | %N |
|---|---|---|---|
| Calculé | 62,17 | 5,74 | 7,25 |
| Trouvé | 61,90 | 5,43 | 6,99 |

Spectre I.R. (KBr) ν($cm^{-1}$): 3310, 2980, 1705, 1670, 1540, 1520, 1500, 1250, 1200, 1120, 1100, 1020, 830, 780, 750, 710.

Spectre R.M.N. (δ, DMSO-$d_6$) p.p.m.: 9,7 (1H, s); 8,9 (1H, s); 7,7 à 6,6 (8H, m); 4,3 à 3,3 (9H, m); 2,2 (6H, s); 2,0 (3H, s); 1,8 à 1,3 (4H, m).

Exemple 25

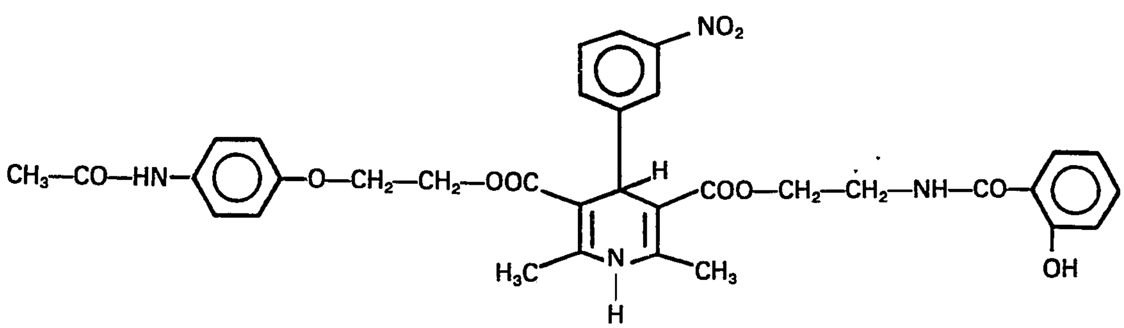

On chauffe à reflux pendant 8 heures une solution de 13,05 g (0.05 mol) de 3-aminocrotonate de 2-(N-salicylamido)éthyle et 20,36 g (0,05 mol) de 2-(3-nitrobenzylidène)acétylacétate de 2-(4-acétylaminophénoxy)éthyle dans 50 ml d'éthanol absolu. Après le refroidissement du mèlange de réaction à −5°C, on obtient le 5-[2-(4-acétylaminophénoxy)éthoxycarbonyl]-2,6-diméthyl-4-(3-nitrophényl)-1,4-dihydropyridine-3-carboxylate de 2-(N-salicylamido)éthyle, sous la forme de cristaux jaunes légèrement hygroscopiques fondant à 112—121°C (recristallisé dans éthanol absolu); le rendement est de 66% de la théorie.

| Analyse pour $C_{34}H_{34}N_4O_{10}$: | %C | %H | %N |
|---|---|---|---|
| Calculé | 62,00 | 5,20 | 8,51 |
| Trouvé | 62,33 | 4,87 | 8,46 |

Spectre I.R. (KBr) ν($cm^{-1}$): 3360, 2960, 1700, 1650, 1600, 1540, 1520, 1490, 1350, 1250, 1210, 1130, 1100, 1020, 830, 780, 755, 715.

Spectre R.M.N. (δ, $CDCl_3$ + DMSO-$d_6$) p.p.m.: 12,2 (1H, s); 9,4 (1H, s); 8,6 à 6,5 (14H, m); 5 (1H, s); 4,4 à 3,8 (6H, m); 3,8 à 3,3 (2H, m); 2,3 (6H, s); 2,05 (3H, s).

Exemple 26

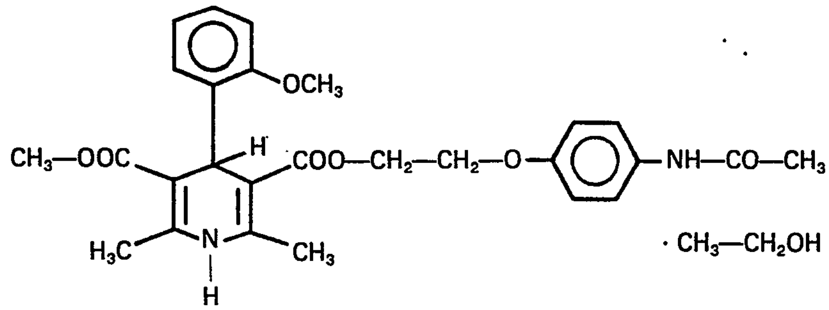

On chauffe à reflux pendant 8 heures une solution de 15 g (0,05 mol) de 3-aminocrotonate de 2-(4-acétylaminophénoxy)éthyle et 12,63 g (0,05 mol) de 2-(2-méthoxybenzylidène)acétylacétate de méthyle dans 60 ml d'éthanol. Après le refroidissement du mélange de réaction à −5°C, on obtient le 2,6-diméthyl-5-méthoxycarbonyl-4-(2-mèthoxyphényl)-1,4-dihydropyridine-3-carboxylate de 2-(4-acétylaminophénoxy)-éthyle, avec une molécule d'éthanol, sous la forme de poudre blanche légèrement jaune fondant à 98—101°C (recristallisé dans éthanol); le rendement est de 55% de la théorie.

| Analyse pour $C_{27}H_{30}N_2O_7.C_2H_6O$: | %C | %H | %N |
|---|---|---|---|
| Calculé | 64,43 | 6,71 | 5,18 |
| Trouvé | 64,66 | 7,00 | 5,19 |

Spectre I.R. (KBr) ν($cm^{-1}$): 3360, 3100, 2960, 1685, 1520, 1495, 1310, 1245, 1210, 1120, 1020, 830, 760.

Spectre R.M.N. (δ, $CDCl_3$) p.p.m.: 8,4 (1H, s); 7,5 à 6,5 (10H, m); 5,3 (1H, s); 4,4 à 3,9 (4H, m); 3,8 à 3,4 (3H + 3H + 2H, s + sd); 2,2 à 2,05 (6H + 3H, s + s); 1,2 (3H, t).

Exemple 27

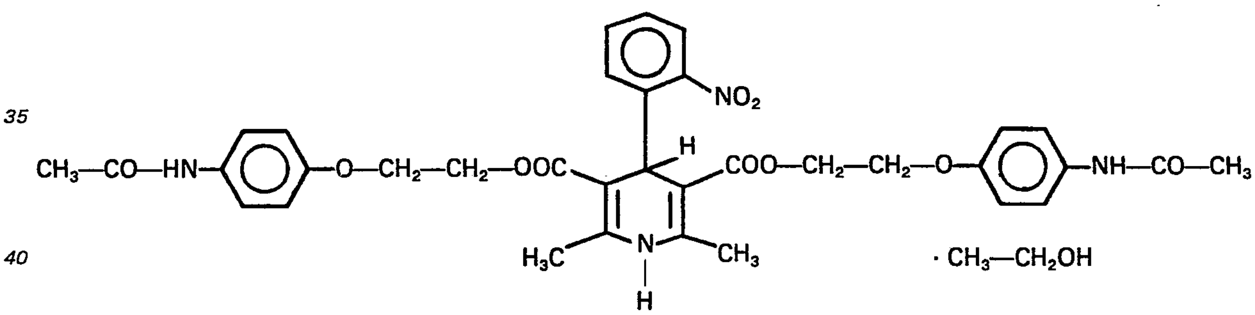

On chauffe à reflux pendant 2 heures une solution de 20 g (0,05 mol) de 2-(2-nitrobenzylidène)acétylacétate de 2-(4-acétylaminophénoxy)éthyle et 13,5 g (0,05 mol) de 3-aminocrotonate de 2-(4-acétylaminophénoxy)éthyle dans 50 ml d'éthanol. Après le refroidissement du mèlange de réaction à la température ambiante, on obtient le 2,6-diméthyl-4-(2-nitrophényl)-1,4-dihydropyridine-3,5-carboxylate de bis-2-(4-acétylaminophénoxy)éthyle, avec une molécule d'éthanol, sous la forme de cristaux jaunes fondant à 148—154°C (recristallisé dans éthanol); le rendement est de 46% de la théorie.

| Analyse pour $C_{35}H_{36}N_4O_{10}.C_2H_6O$: | %C | %H | %N |
|---|---|---|---|
| Calculé | 61,83 | 5,89 | 7,79 |
| Trouvé | 61,49 | 6,07 | 7,85 |

Spectre I.R. (KBr) ν($cm^{-1}$): 3330, 3300, 3100, 1710, 1680, 1540, 1520, 1350, 1250, 1210, 1105, 935, 840, 715.

Spectre R.M.N. (δ, DMSO-$d_6$) p.p.m.: 9,7 (1H, sd); 8,9 (1H, sl); 7,7 à 6,5 (14H, m); 5.65 (1H, sd); 4,4 à 3,85 (8H, sd); 3,4 (2H, m); 2,4 à 1,9 (6H + 6H, s + s); 1,1 (3H, t).

Exemple 28

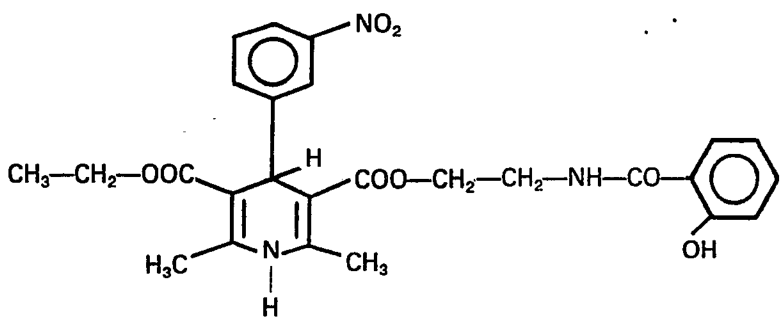

On chauffe à reflux pendant 8 heures une solution de 15 g (0,06 mol) de 3-aminocrotonate de 2-(N-salicylamido)éthyle et 14,94 g (0,06 mol) de 2-(3-nitrobenzylidène)acétylacétate d'éthyle dans 55 ml d'éthanol. Après l'évaporation du solvant, addition de 5 ml de méthanol et refroidissement à −5°C du mélange résultant, on obtient le 2,6-diméthyl-5-éthoxycarbonyl-4-(3-nitrophényl)-1,4-dihydropyridine-3-carboxylate de 2-(N-salicylamido)éthyle, sous la forme de poudre jaune fondant à 127—130°C; le rendement est de 86% de la théorie.

| Analyse pour $C_{26}H_{27}N_3O_8$: | %C | %H | %N |
|---|---|---|---|
| Calculé | 61,29 | 5,34 | 8,25 |
| Trouvé | 61,28 | 5,34 | 8,24 |

Spectre I.R. (KBr) ν(cm$^{-1}$): 3460, 3400, 3000, 1700, 1660, 1540, 1490, 1350, 1300, 1220, 1125, 1095, 1030, 780, 750, 700.

Spectre R.M.N. (δ, $CDCl_3$) p.p.m.: 12 (1H, sd); 8,2 à 6,5 (10H, m); 5,1 (1H, s); 4,4 à 3,4 (4H + 2H, m); 2,3 (6H, s); 1,2 (3H, t).

Exemple 29

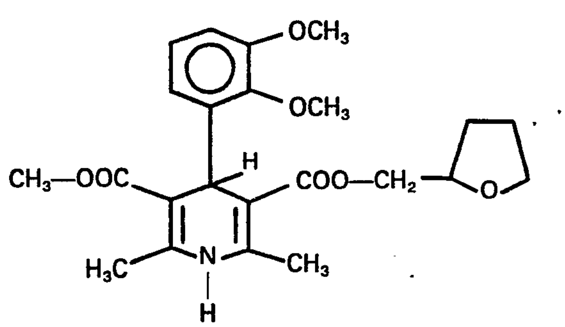

On chauffe à reflux pendant 8 heures une solution de 10 g (0,04 mol) de 2-(2,3-diméthoxybenzylidène)-acétylacétate de méthyle et 7,01 g (0,04 mol) de 3-aminocrotonate de 2-tétrahydrofurfuryle dans 40 ml d'éthanol. Après l'évaporation du solvant, dissolution de l'huile résultant dans 10 ml d'acétate d'éthyle à ébullition et refroidissement à −5°C, on obtient le 4-(2,3-diméthoxyphényl)-2,6-diméthyl-5-méthoxycarbonyl-1,4-dihydropyridine-3-carboxylate de 2-tétrahydrofurfuryle, sous la forme de prismes blancs fondant à 138—140°C (recristallisé dans éthanol); le rendement est de 48% de la théorie.

| Analyse pour $C_{23}H_{29}NO_7$: | %C | %H | %N |
|---|---|---|---|
| Calculé | 64,02 | 6,77 | 3,25 |
| Trouvé | 63,75 | 6,75 | 3,40 |

Spectre I.R. (KBr) ν(cm$^{-1}$): 3320, 3260, 2960, 1700, 1640, 1520, 1480, 1310, 1290, 1210, 1100, 1070, 1020, 810, 740.

Spectre R.M.N. (δ, $CDCl_3$) p.p.m.: 6,9 (4H, sd); 5,3 (1H, s); 4,4 à 3,6(5H + 6H + 3H, m + s + s); 2,2 (6H, s); 2,1 à 1,7 (4H, m).

Exemple 30

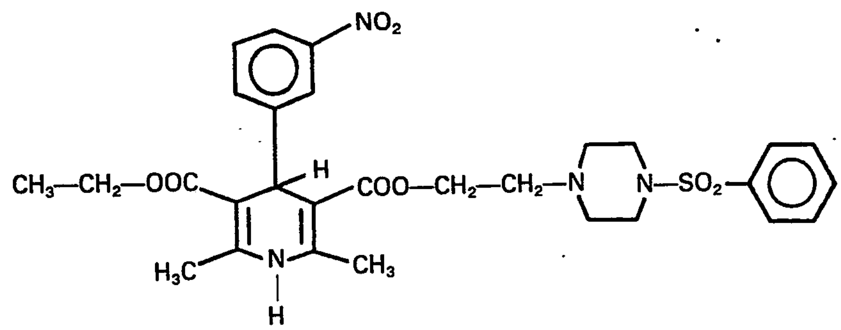

On chauffe à reflux pendant 8 heures une solution de 5,02 g (0,02 mol) de 2-(3-nitrobenzylidène)acétylacétate d'éthyle et 6,74 g (0,02 mol) 3-aminocrotonate de 2-(4-benzènesulfonyl-1-pipérazinyl)éthyle dans 20 ml d'éthanol. Après l'évaporation du solvant, on obtient le 2,6-diméthyl-5-éthoxycarbonyl-4-(3-nitrophényl)-1,4-dihydropyridine-3-carboxylate de 2-(4-benzènesulfonyl-1-pipérazinyl)éthyle sous la forme de poudre jaune fondant à 161—163°C (recristallisé dans éthanol); le rendement est de 76% de la théorie.

| Analyse pour $C_{29}H_{34}N_4O_8S$: | %C | %H | %N | %S |
|---|---|---|---|---|
| Calculé | 58,18 | 5,72 | 9,36 | 5,36 |
| Trouvé | 57,89 | 5,63 | 9,42 | 5,72 |

Spectre I.R. (KBr) ν($cm^{-1}$): 3400, 3120, 2980, 2830, 1710, 1660, 1540, 1495, 1355, 1220, 1170, 1110, 950, 740, 690.

Spectre R.M.N. (δ, $CDCl_3$) p.p.m.: 8 à 7,1 (9H, m); 6,3 (1H, s); 5 (1H, s); 4,2 à 3,8 (4H, m); 3,1 à 2,3 (10H, m); 2,3 (6H, s); 1,2 (3H, t).

Exemple 31

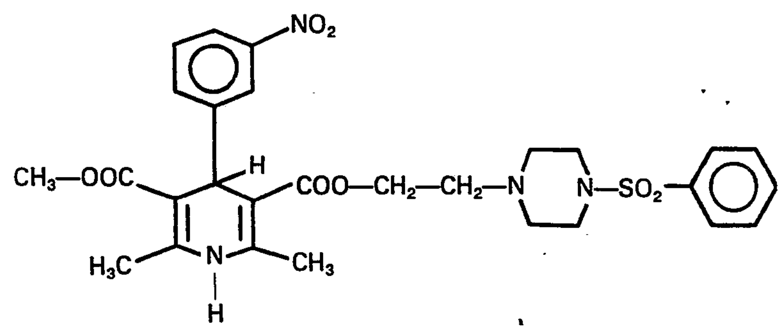

On chauffe à reflux pendant 8 heures une solution de 4,94 g (0,02 mol) de 2-(3-nitrobenzylidène)-acétylacétate de méthyle et 7,0 g (0,02 mol) de 3-aminocrotonate de 2-(4-benzènesulfonyl-1-pipérazinyl)-éthyle dans 20 ml d'éthanol. Après le refroidissement du mélange de réaction à −5°C, on obtient le 2,6-diméthyl-5-méthoxycarbonyl-4-(3-nitrophényl)-1,4-dihydropyridine-3-carboxylate de 2-(4-benzènesulfonyl-1-pipérazinyl)éthyle, sous la forme de cristaux jaunes légèrement hygroscopiques fondant à 106—120°C avec décomposition (recristallisé dans éthanol absolu); le rendment est de 65% de la théorie.

| Analyse pour $C_{28}H_{32}N_4O_8S$: | %C | %H | %N | %S |
|---|---|---|---|---|
| Calculé | 57,52 | 5,52 | 9,58 | 5,48 |
| Trouvé | 57,45 | 5,75 | 9,62 | 5,53 |

Spectre I.R. (KBr) ν($cm^{-1}$): 3360, 2940, 2840, 1705, 1660, 1530, 1485, 1350, 1220, 1170, 1120, 1015, 950, 740, 700, 690.

Spectre R.M.N. (δ, $CDCl_3$) p.p.m.: 8,1 à 7,0 (9H, m); 6,7 (1H, s); 5 (1H, s); 4,1 (2H, td); 3,6 (3H, s); 3,2 à 2,2 (10H + 6H, m + s).

Exemple 32

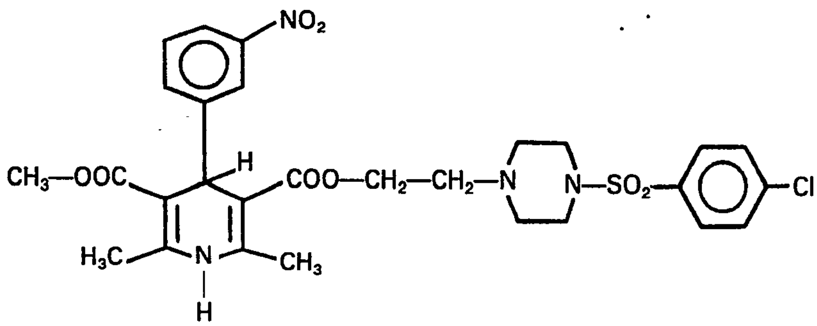

On chauffe à reflux pendant 8 heures une solution de 1,11 g (2,86 × $10^{-3}$ mol) de 3-aminocrotonate de 2-[4-(4-chlorobenzènesulfonyl)-1-pipérazinyl]éthyle et 0,72 g (2,86 × $10^{-3}$ mol) de 2-(3-nitrobenzylidène)-acétylacétate de méthyle dans 10 ml d'éthanol. Après l'évaporation du solvant, addition de 3 ml de méthanol et refroidissement à −5°C, on obtient le 2,6-diméthyl-5-méthoxycarbonyl-4-(3-nitrophényl)-1,4-dihydropyridine-3-carboxylate de 2-[4-(4-chlorobenzènesulfonyl)-1-pipérazinyl]éthyle, sous la forme de cristaux jaunes pâles fondant à 97—105°C avec décomposition; le rendement est de 69% de la théorie.

| Analyse pour $C_{28}H_{31}ClN_4O_8S$: | %C | %H | %N | %Cl | %S |
|---|---|---|---|---|---|
| Calculé | 54,32 | 5,05 | 9,05 | 5,73 | 5,18 |
| Trouvé | 53,97 | 5,20 | 9,26 | 5,83 | 6,32 |

Spectre I.R. (KBr) ν($cm^{-1}$): 3360, 3080, 2940, 2810, 1700, 1530, 1480, 1350, 1210, 1170, 1090, 1010, 950, 820, 760, 700

Spectre R.M.N. (δ, $CDCl_3$) p.p.m.: 8,1 à 7,2 (8H, m); 6,72 (1H, s); 5,1 (1H, s);, 4,1 (2H, td); 3,6 (3H, sd); 3,2 à 2,1 (10H + 6H, m + s).

Exemple 33

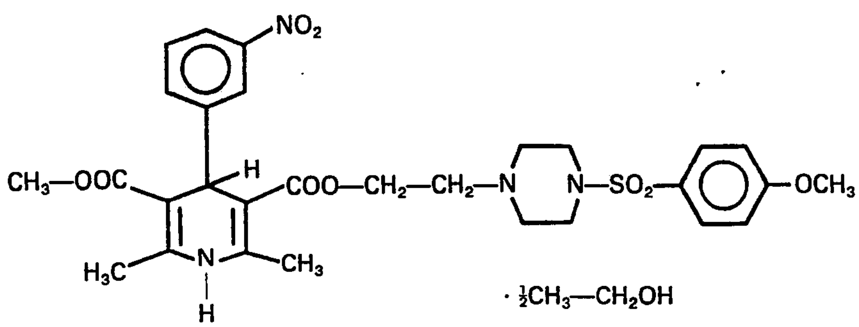

$\cdot \frac{1}{2}CH_3—CH_2OH$

On chauffe à reflux pendant 8 heures une solution de 6,41 g (0,02 mol) de 3-aminocrotonate de 2-[4-(4-méthoxybenzènesulfonyl)-1-pipérazinyl]éthyle et 4,17 g (0.02 mol) de 2-(3-nitrobenzylidène)acétylacétate de méthyle dans 20 ml d'éthanol. Après l'évaporation du solvant et addition de 5 ml de méthanol à ébullition, on obtient le 2,6-diméthyl-5-méthoxycarbonyl-4-(3-nitrophényl)-1,4-dihydropyridine-3-carboxylate de 2-[4-(4-méthoxybenzènesulfonyl)-1-pipérazinyl]éthyle avec une demie molécule d'éthanol, sous la forme de cristaux jaunes fondant à 82—104°C avec décomposition (recristallisé dans éthanol); le rendement est de 67% de la théorie.

| Analyse pour $C_{29}H_{34}N_4O_9S.\frac{1}{2}\ C_2H_6O$: | %C | %H | %N | %S |
|---|---|---|---|---|
| Calculé | 56,50 | 5,85 | 8,79 | 5,03 |
| Trouvé | 56,66 | 6,00 | 9,30 | 5,93 |

Spectre I.R. (KBr) ν($cm^{-1}$): 3360, 2940, 2830, 1700, 1590, 1530, 1500, 1350, 1220, 1160, 1090, 800, 730, 700.

Spectre R.M.N. (δ, $CDCl_3$) p.p.m.: 8,05 à 6,7 (9H, m); 5,05 (1H, s); 4,2 à 3,3 (2H + 3H + 3H + 1H, td + s + s + m); 3,1 à 2,2 (10CH + 6H, m + s); 1,2 ($\frac{1}{2}$3H, t).

Exemple 34

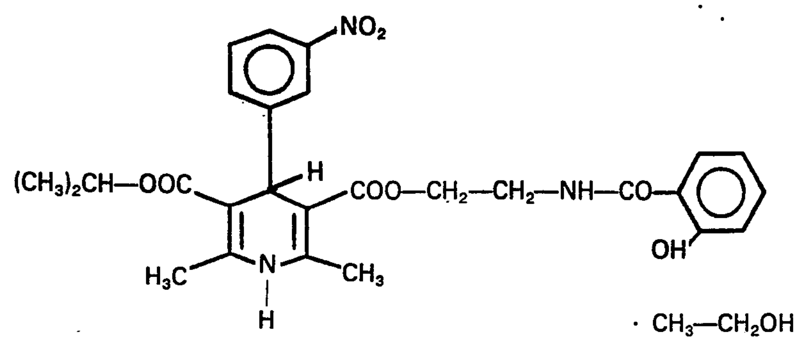

On chauffe à reflux pendant 8 heures une solution de 15 g (0,05 mol) de 2-(3-nbitrobenzylidène)acétylacétate d'isopropyle et 14,30 g (0,05 mol) de 3-aminocrotonate de 2-(N-salicylamido)éthyle dans 55 ml d'éthanol. Après l'évaporation du solvant, addition de 15 ml d'acétate d'éthyle à ébullition et refroidissement à 7°C, on obtient le 2,6-diméthyl-5-isopropoxycarbonyl-4-(3-nitrophényl)-1,4-dihydropyridine-3-carboxylate de 2-(N-salicylamido)éthyle avec une molécule d'éthanol, sous la forme d'aiguilles jaunes fondant à 107—113°C avec décomposition (recristallisé dans éthanol); le rendement est de 79% de la théorie.

| Analyse pour $C_{27}H_{29}N_3O_8.C_2 + H_6$: | %C | %H | %N |
|---|---|---|---|
| Calculé | 61,15 | 6,19 | 7,38 |
| Trouvé | 61,45 | 6,07 | 7,62 |

Spectre I.R. (KBr) ν($cm^{-1}$): 3340, 3080, 2960, 1680, 1660, 1640, 1530, 1490, 1345, 1300, 1210, 1100, 1010, 775, 745, 695.

Spectre R.M.N. (δ, DMSO-$d_6$) p.p.m.: 12,7 (1H, s); 9 (2H, m); 8,3 à 6,8 (8H, m); 5,2 (1H, s); 4,95 (1H, m); 4,3 (2H, td); 3,6 (4H, m); 2,4 (6H, s); 1,1 (9H, t).

Exemple 35

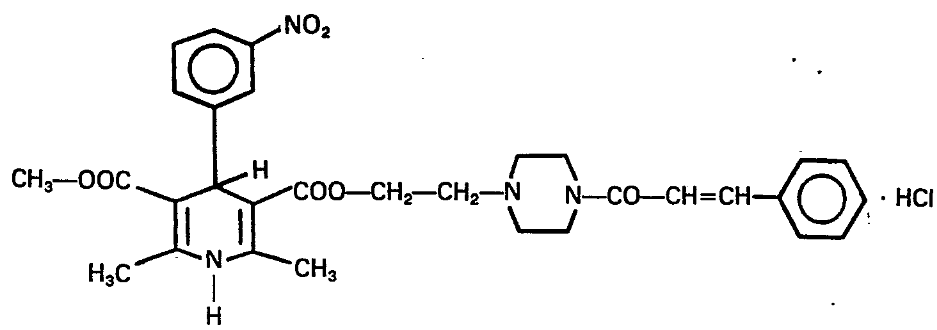

On chauffe à reflux pendant 8 heures une solution de 15 g (0,06 mol) de 2-(3-nitrobenzylidène)acétylacétate de méthyle et 20,67 g (0,06 mol) de 3-aminocrotonate de 2-(4-cinnamoyl-1-pipérazinyl)éthyle dans 60 ml d'éthanol. Après le refroidissement à la température ambiante, on obtient le chlorhydrate par addition de 1,75 ml d'une solution saturée de chlorure d'hydrogène dans éther éthylique. Après évaporation du solvant, on sèche le résidu sous vide dans un dessicateur avec $CaCl_2$, pour rendre ainsi le chlorhydrate de 2,6-diméthyl-5-méthoxycarbonyl-4-(3-nitrophényl)-1,4-dihydropyridine-3-carboxylate de 2-(4-cinnamoyl-1-pipérazinyl)éthyle, sous la forme de cristaux jaunes écumeux fondant à 90—150°C avec décomposition; le rendement est de 67% de la théorie.

| Analyse pour $C_{31}H_{34}N_4O_7.HCl$: | %C | %H | %N | %Cl |
|---|---|---|---|---|
| Calculé | 60,93 | 5,77 | 9,17 | 5,80 |
| Trouvé | 60,73 | 5,64 | 8,82 | 5,52 |

Spectre I.R. (KBr) ν($cm^{-1}$): 3250, 3080, 2950, 1700, 1650, 1530, 1400, 1430, 1350, 1210, 1115, 1100, 1020, 760, 700.

Spectre R.M.N. (δ, $CDCl_3$) p.p.m.: 8 à 6,6 (12H, m); 5,2 (1H, s); 5 (1H, s); 4,4 (2H, m); 4 à 3,6 (4H + 3H, m + s); 3 (6H, m); 2,35 (6H, sd).

Exemple 36

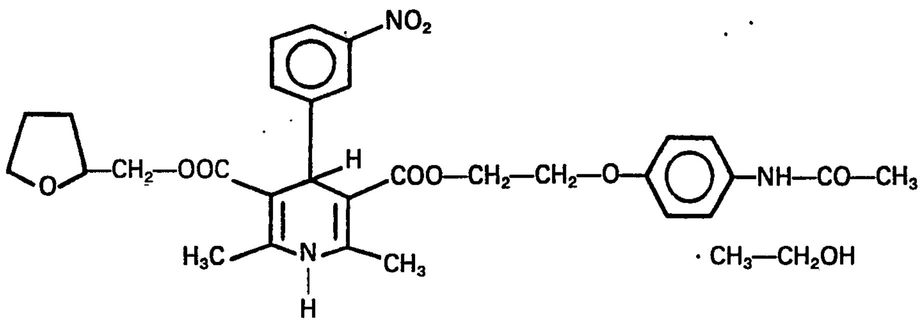

On chauffe à reflux pendant 8 heures une solution de 20 g (0.06 mol) de 2-(3-nitrobenzylidène)acétylacétate de 2-tétetrahydrofuryle et 17,43 g (0,06 mol) de 3-aminocrotonate de 2-(4-acétylaminophénoxy)-éthyle dans 60 ml d'éthanol. Après l'évaporation du solvant, addition de 25 ml d'acétate d'éthyle à ébullition et refroidissement à 7°C, on obtient le 2,6-diméthyl-4-(4-nitrophényl)-5-(2-tétrahydrofurfuryl-oxy-carbonyl)-1,4-dihydropyridine-3-carboxylate de 2-(4-acétylaminophénoxy)éthyle, avec une molécule d'éthanol, sous la forme de poudre jaune fondant à 88—92°C avec décomposition (recristallisé dans acétate d'éthyle); le rendement est de 46% de la théorie.

| Analyse pour $C_{30}H_{33}N_3O_9.C_2H_6O$: | %C | %H | %N |
|---|---|---|---|
| Calculé | 61,43 | 6,38 | 6,72 |
| Trouvé | 61,22 | 6,24 | 6,43 |

Spectre I.R. (KBr) ν($cm^{-1}$): 3410, 3350, 2950, 2880, 1700, 1670, 1535, 1350, 1250, 1210, 1120, 1020, 830, 750, 710.

Spectre R.M.N. (δ, $CDCl_3$) p.p.m.: 8,4 à 6,6 (10H, m); 5,1 (1H, s); 4,6 à 3,5 (11H, s); 2,35 (6H, s); 2,2 à 1,5 (7H, m); 1,25 (3H, t).

Exemple 37

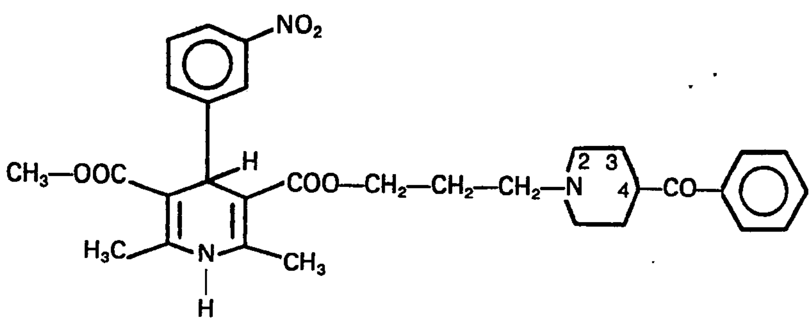

On chauffe à reflux pendant 8 heures une solution de 9,18 g (0,03 mol) de 3-aminocrotonate de 3-(4-benzoyl-1-pipéridinyl)propyle et 6,92 g (0,03 mol) de 2-(3-nitrobenzylidène)acétylacétate de méthyle dans 30 ml d'éthanol. Après l'évaporation du solvant, addition de 10 ml de méthanol à ébullition et refroidissement à −5°C, on obtient le 2,6-diméthyl-5-méthoxycarbonyl-4-(3-nitrophényl)-1,4-dihydropyridine-3-carboxylate de 3-(4-benzoyl-1-pipéridinyl)propyle, sous la forme de cristaux jaunes fondant à 133—136°C (recristallisé dans éthanol); le rendement est de 26% de la théorie.

| Analyse pour $C_{31}H_{35}N_3C_7$: | %C | %H | %N |
|---|---|---|---|
| Calculé | 66,30 | 6,28 | 7,48 |
| Trouvé | 65,97 | 6,56 | 7,18 |

Spectre I.R. (KBr) ν($cm^{-1}$): 3380, 2960, 1705, 1680, 1660, 1530, 1485, 1350, 1220, 1120, 1100, 980, 780, 745, 700.

Spectre R.M.N. (δ, $CDCl_3$) p.p.m.: 8.1 à 7 (10H, m); 5,1 (1H, s); 4,1 (2H, m); 3,6 (3H, s); 3,5 à 1,5 (5H + 6H + 8H).

Exemple 38

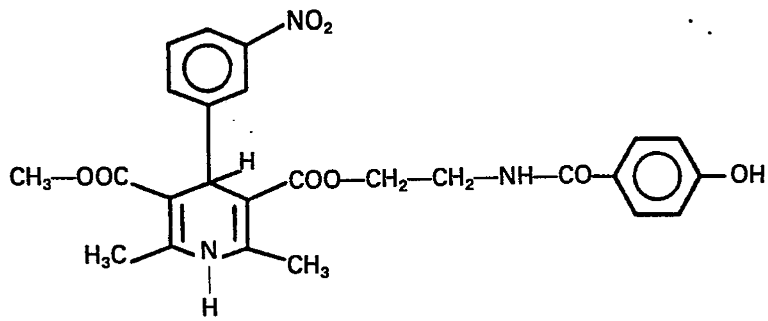

On chauffe à reflux pendant 8 heures une solution de 10 g (0,04 mol) de 3-aminocrotonate de 2-[N-(4-hydroxybenzoyl)amino]éthyle et 9,43 g (0,04 mol) de 2-(3-nitrobenzylidène) acétylacétate de méthyle dans 40 ml d'éthanol. Après l'évaporation du solvant, on obtient le 2,6-diméthyl-5-méthoxycarbonyl-4-(3-nitrophényl)-1,4-dihydropyridine-3-carboxylate de 2-[N-(4-hydroxybenzoyl)amino]éthyle, sous la forme de cristaux jaunes écumeux fondant à 110—120°C avec décomposition; le rendement est de 96% de la théorie.

| Analyse pour $C_{25}H_{25}N_3O_8$: | %C | %H | %N |
|---|---|---|---|
| Calculé | 60,60 | 5,08 | 8,48 |
| Trouvé | 60,26 | 5,05 | 8,72 |

Spectre I.R. (KBr) ν($cm^{-1}$): 3360, 2940, 1700, 1650, 1530, 1510, 1350, 1210, 1115, 1090, 1020, 840, 760, 700.

Spectre R.M.N. (δ, $CDCl_3$ + DMSO-$d_6$) p.p.m.: 9,4 (1H, s); 8,3 à 6,7 (10H, m); 5,05 (1H, s); 4,2 (2H, t); 3,6 (2H + 3H, m + s); 2,35 (6H, s).

Exemple 39

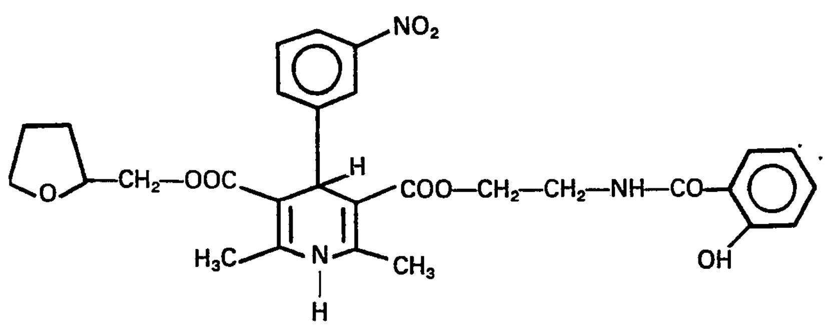

On chauffe à reflux pendant 8 heures une solution de 15 g (0.05 mol) de 2-(3-nitrobenzylidène)-acétylacétate de 2-tétrahydrofurfuryle et 12,41 g (0,05 mol) de 3-aminocrotonate de 2-(N-salicylamido)-éthyle dans 50 ml d'éthanol. Après l'évaporation du solvant, l'huile résultant est dissoute dans 30 ml d'éthanol aqueux à 50% à ébullition et refroidit à −5°C. Puis on sépare par décantation le produit insoluble, on le sèche sous vide dans un dessicateur avec $CaCl_2$ pour obtenir ainsi le 2,6-diméthyl-4-(3-nitrophényl)-5-(2-tétrahydrofurfuryloxycarbonyl)-1,4-dihydropyridine-3-carboxylate de 2-(N-salicylamido)-éthyle, sous la forme de poudre jaune fondant à 115—125°C avec décomposition (recristallisé dans éthanol); le rendement est de 66% de la théorie.

| Analyse pour $C_{29}H_{31}N_3O_9$: | %C | %H | %N |
|---|---|---|---|
| Calculé | 61,59 | 5,52 | 7,43 |
| Trouvé | 61,50 | 5,22 | 7,51 |

Spectre I.R. (KBr) ν($cm^{-1}$): 3440, 3360, 2960, 2860, 1700, 1650, 1530, 1490, 1340, 1220, 1120, 1015, 820, 775, 750, 690.

Spectre R.M.N. (δ, $CDCl_3$) p.p.m.: 11,9 (1H, sl); 8,2 à 6,6 (10H, m); 5,1 (1H, s); 4,4 à 3,4 (9H, m); 2,35 (6H, s); 2,1 à 1,5 (4H, m).

Exemple 40

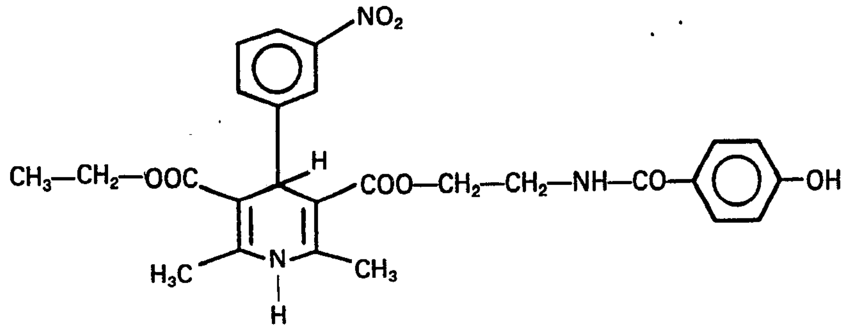

On chauffe à reflux pendant 8 heures une solution de 11 g (0,04 mol) de 3-aminocrotonate de 2-[N-(4-hydroxybenzoyl)amino]éthyle et 10,96 g (0.04 mol) de 2-(3-nitrobenzylidène)acétylacétate d'éthyle dans 45 ml d'éthanol. Après décoloration avec charbon actif et l'évaporation du solvant, on obtient le 2,6-diméthyl-5-éthoxycarbonyl-4-(3-nitrophényl)-1,4-dihydropyridine-3-carboxylate de 2-[N-(4-hydroxybenzoyl)amino]-éthyle, sous la forme de cristaux jaunes écumeux fondant à 107—116°C avec décomposition; le rendement est de 72% de la théorie.

| Analyse pour $C_{26}H_{27}N_3O_8$: | %C | %H | %N |
|---|---|---|---|
| Calculé | 61,29 | 5,34 | 8,25 |
| Trouvé | 61,98 | 5,51 | 8,25 |

Spectre I.R. (KBr) ν($cm^{-1}$): 3350, 3080, 2980, 1690, 1645, 1610, 1530, 1505, 1345, 1210, 1120, 1015, 840, 760, 740, 700.

Spectre R.M.N. (δ, $CDCl_3$ + DMSO-$d_6$) p.p.m.: 8,4 à 6,6 (11H, m); 5 (1H, s); 4,3 à 3,8 (4H, m); 3,5 (2H, m); 2,3 (6H, s); 1,15 (3H, t).

Exemple 41

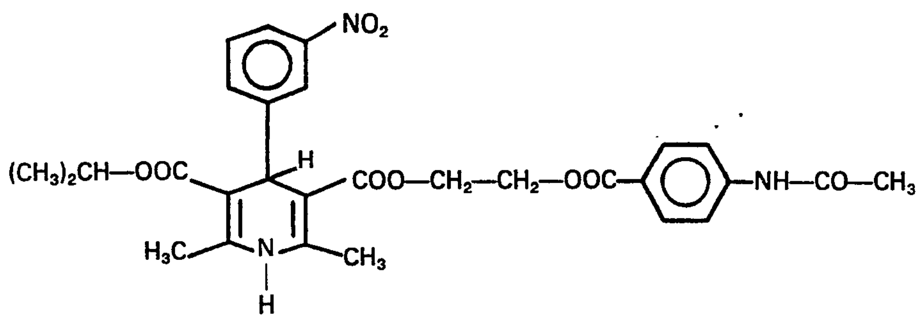

On chauffe à reflux pendant 8 heures une solution de 10 g (0,02 mol) de 2-(3-nitrobenzylidène)-acétylacétate de 2-(4-acétylaminobenzoyloxy)éthyle et 3,25 g (0.02 mol) de 3-aminocrotonate d'isopropyle dans 35 ml d'éthanol. Après le refroidissement du mélange de réaction à −5°C, on obtient le 2,6-diméthyl-5-isopropoxycarbonyl-4-(3-nitrophényl)-1,4-dihydropyridine-3-carboxylate de 2-(4-acétylaminobenzoyloxy)-éthyle, sous la forme de cristaux jaunes fondant à 193—195°C (recristallisé dans éthanol); le rendement est de 65% de la théorie.

| Analyse pour $C_{29}H_{31}N_3O_9$: | %C | %H | %N |
|---|---|---|---|
| Calculé | 61,59 | 5,52 | 7,43 |
| Trouvé | 61,29 | 5,76 | 7,37 |

Spectre I.R. (KBr) ν($cm^{-1}$): 3400, 3320, 3100, 2980, 1705, 1605, 1540, 1500, 1410, 1360, 1260, 1210, 1095, 850, 769, 745, 710, 690.

Spectre R.M.N. (δ, DMSO-$d_6$) p.p.m.: 10,4 (1H, s); 9,1 (1H, s); 8,3 à 7,4 (9H, m); 5,2 à 4,8 (1H + 1H, s + m); 4,45 (:4H, sl); 2,4 (6H, s); 2,2 (3H, s); 1,15 (6H, t).

Exemple 42

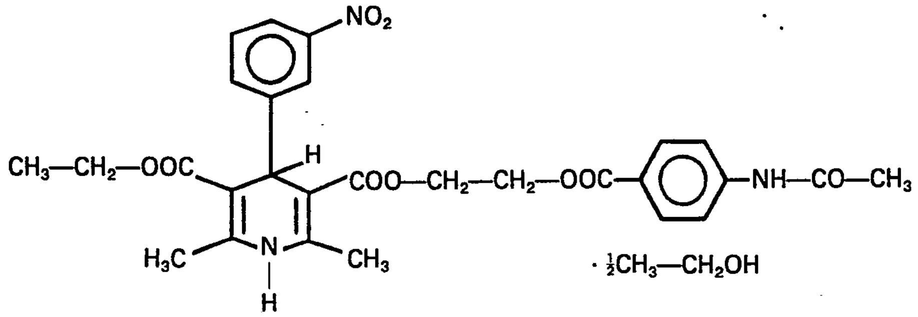

On chauffe à reflux pendant 8 heures une solution de 20 g (0,07 mol) d'acétylacétate de 2-(4-acétylaminobenzoyloxy) éthyle, 8,41 g (0,07 mol) de 3-aminocrotonate d'éthyle et 9,84 g (0,07 mol) de 3-nitrobenzaldéhyde dans 65 ml d'éthanol. Après l'évaporation du solvant, addition de 15 ml de méthanol à ébullition, décoloration avec charbon actif et refroidissement à −5°C, on obtient le 2,6-diméthyl-5-éthoxy-carbonyl-4-(3-nitrophényl)-1,4-dihydropyridine-3-carboxylate de 2-(4-acétylaminobenzoyloxy)éthyle avec une demie molécule d'éthanol, sous le forme de cristaux jaunes fondant à 157—162°C (recristallisé dans éthanol); le rendement est de 46% de la théorie.

| Analyse pour $C_{28}H_{29}N_3O_9.\frac{1}{2}\ C_2H_6O$: | %C | %H | %N |
|---|---|---|---|
| Calculé | 60,62 | 5,61 | 7,31 |
| Trouvé | 60,17 | 5,86 | 7,52 |

Spectre I.R. (KBr) ν(cm$^{-1}$): 3360, 3250, 2990, 1720, 1690, 1660, 1530, 1475, 1345, 1270, 1210, 1110, 1090, 1050, 760, 745, 700.

Spectre R.M.N. (δ, DMSO-$d_6$) p.p.m.: 10.4 (1H, s); 9,1 (1H, s); 8,2 à 7,3 (8H, m); 5,15 (1H, s); 4,6 à 3,8 (7H, m); 2,4 (6H, s); 2,15 (3H, s); 1,1 (4,5 t).

Exemple 43

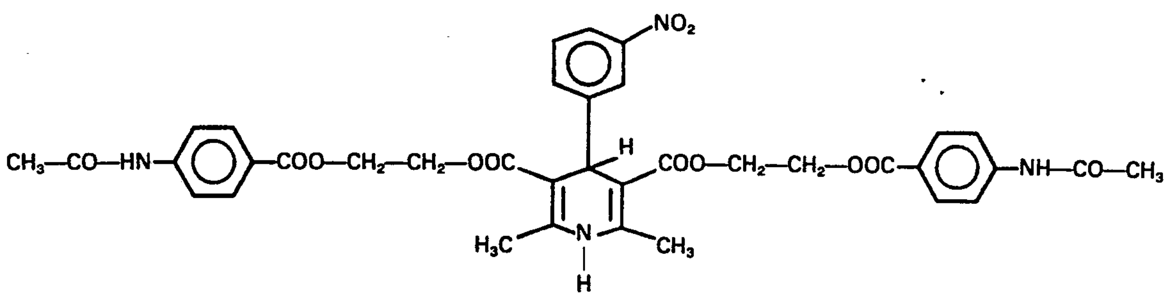

On chauffe à reflux pendant 8 heures une solution de 13,27 g (0.03 mol) de 2-(3-nitrobenzylidène)-acétylacétate de 2-(4-acétylaminobenzoyloxy)éthyle, 9,26 g (0,03 ml) d'acétylacétate de 2-(4-acétylamino-benzoyloxy)éthyle et 5 ml d'une solution aqueuse concentrée d'hydroxyde d'ammonium dans 50 ml d'éthanol. Après l'évaporation de 30 ml du solvant et refroidissement à −5°C du mélange de réaction, on obtient le 2,6-diméthyl-4-(3-nitrophényl)-1,4-dihydropyridine-3,5-dicarboxylate de bis-2-(4-acétylamino-benzoyloxy)éthyle, sous la forme de cristaux jaunes fondant à 147—150°C (recristallisé dans éthanol); le rendement est de 46% de la théorie.

| Analyse pour $C_{37}H_{36}N_4O_{12}$: | %C | %H | %N |
|---|---|---|---|
| Calculé | 66,99 | 4,98 | 7,69 |
| Trouvé | 60,61 | 5,15 | 7,91 |

Spectre I.R. (KBr) ν(cm$^{-1}$): 3320, 3100, 2940, 1720, 1700, 1680, 1600, 1540, 1490, 1410, 1350, 1270, 1200, 1100, 850, 760, 710.

Spectre R.M.N. (δ, $CDCl_3$ + DMSO-$d_6$) p.p.m.: 9,9 (2H, s); 8,7 (1H, s); 8,2 à 7(12H, m); 5 (1H, s); 4,3 (8H, s); 2,3 (6H, s); 2,1 (6H, s).

Exemple 44

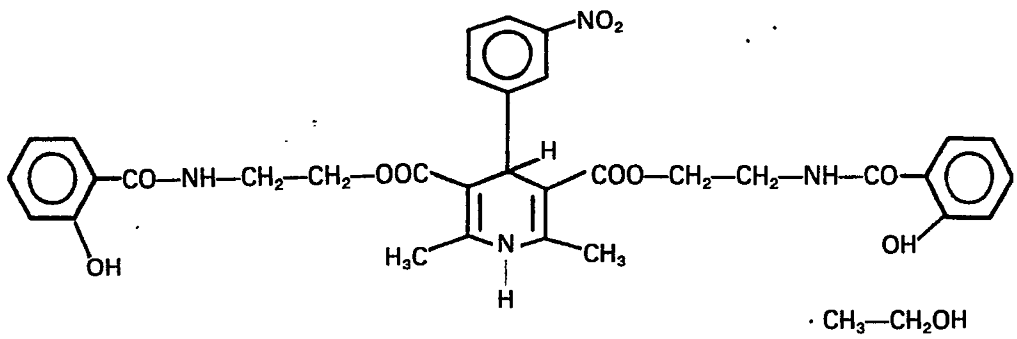

$\cdot CH_3—CH_2OH$

On chauffe à reflux pendant 8 heures une solution de 15 g (0,04 mol) de 2-(3-nitrobenzylidène)-acétylacétate de 2-(N-salicylamido)éthyle et 9,95 g (0,04 mol) de 3-aminocrotonate de 2-(N-salicylamido)-éthyle dans 40 ml d'éthanol. Après l'évaporation du solvant, on dissout l'huile résultant dans 200 ml de méthanol et cette solution est décolorée avec charbon actif. L'évaporation finale du solvant donne le 2,6-diméthyl-4-(3-nitrophényl)-1,4-dihydropyridine-3,5-dicarboxylate de bis-2-(N-salicylamido)éthyle avec une molécule d'éthanol, sous la forme de cristaux jaunes écumeux fondant à 107—120°C avec décomposition; le rendement est de 74% de la théorie.

| Analyse pour $C_{33}H_{32}N_4O_{10}.C_2H_6O$: | %C | %H | %N |
|---|---|---|---|
| Calculé | 60,86 | 5,55 | 8,11 |
| Trouvé | 60,46 | 5,42 | 8,33 |

Spectre I.R. (KBr) ν($cm^{-1}$): 3400, 1700, 1640, 1600, 1530, 1490, 1350, 1300, 1210, 1110, 745, 690.

Spectre R.M.N. (δ, $CDCl_3$) p.p.m.: 8 à 6,5 (18H, m); 5,05 (1H, s); 4,2 (4H, m); 3,8 à 3,4 (6H, m); 2,25 (6H, s); 1,2 (3H, t).

Exemple 45

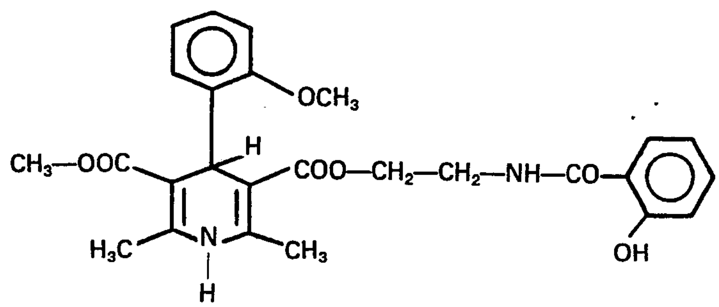

On chauffe à reflux pendant 8 heures une solution de 15 g (0,06 mol) de 3-aminocrotonate de 2-(N-salicylamido)éthyle et 13,30 g (0,06 mol) de 2-(2-méthoxybenzylidène)acétylacétate de méthyle dans 60 ml d'éthanol. Après l'évaporation de 30 ml du solvant et refroidissement à −5°C, on obtient le 2,6-diméthyl-5-méthoxycarbonyl-4-(2-méthoxyphényl)-1,4-dihydropyridine-3-carboxylate de 2-(N-salicylamido)éthyle, sous la forme de cristaux jaunes pâles, fondant à 173—175°C (recristallisé dans éthanol); le rendement est de 45% de la théorie.

| Analyse pour $C_{26}H_{28}N_2O_7$: | %C | %H | %N |
|---|---|---|---|
| Calculé | 64,99 | 5,87 | 5,83 |
| Trouvé | 64,99 | 6,14 | 6,06 |

Spectre I.R. (KBr) ν($cm^{-1}$): 3340, 3360, 2940, 1710, 1640, 1600, 1490, 1300, 1240, 1210, 1110, 1090, 1010, 755.

Spectre R.M.N. (δ, DMSO-$d_6$) p.p.m.: 8,8 (2H, m); 8,1 à 6,7 (9H, m); 5,35 (1H, s); 4,2 (2H, m); 3,75 (3H, s); 3,6 (3H, s); 2,3 (6H, s).

Exemple 46

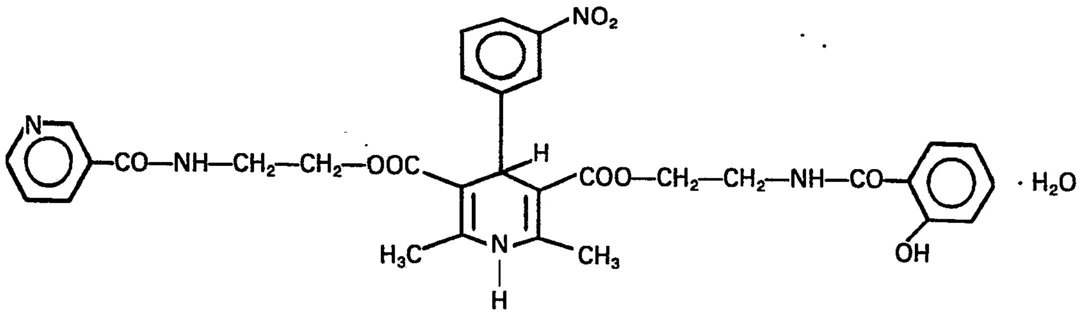

On chauffe à reflux pendant 8 heures une solution de 15 g (0,04 mol) de 2-(3-nitrobenzylidène)acétylacétate de 2-(N-salicylamido)éthyle et 9,39 g (0,04 mol) de 3-aminocrotonate de 2-(N-nicotinoylamino)-éthyle dans 40 ml d'éthanol. La solution résultant est décolorée avec charbon actif et le solvant évaporé. L'huile résultant est cristallisée dans $H_2O$, puis extraite avec $CH_2Cl_2$ (2 × 100 ml) et cette solution séchée sur $Na_2SO_4$ anhydre. L'évaporation finale du solvant donne le 2,6-diméthyl-4-(3-nitrophényl)-1,4-dihydropyridine-5-carboxylate de 2-(N-nicotinoylamino)éthyle-3-carboxylate de 2-(N-salicylamido)éthyle avec une molécule de $H_2O$, sous la forme de cristaux jaunes écumeux, fondant à 88—115°C avec décomposition; le rendement est de 51% de la théorie.

| Analyse pour $C_{32}H_{31}N_5O_9.H_2O$: | %C | %H | %N |
|---|---|---|---|
| Calculé | 59,35 | 5,10 | 10,82 |
| Trouvé | 59,23 | 4,75 | 10,85 |

Spectre I.R. (KBr) ν($cm^{-1}$): 3320, 3060, 2940, 1640, 1600, 1530, 1490, 1350, 1310, 1210, 1120, 1020, 890, 750, 700.

Spectre R.M.N. (δ, DMSO-$d_6$) p.p.m.: 12,2 (1H, s); 8,9 à 6,7 (14H, m); 5 (1H, s); 4,1 (4H, td); 3,5 (4H, m); 3,3 (2H, s); 2,25 (6H, s).

Exemple 47

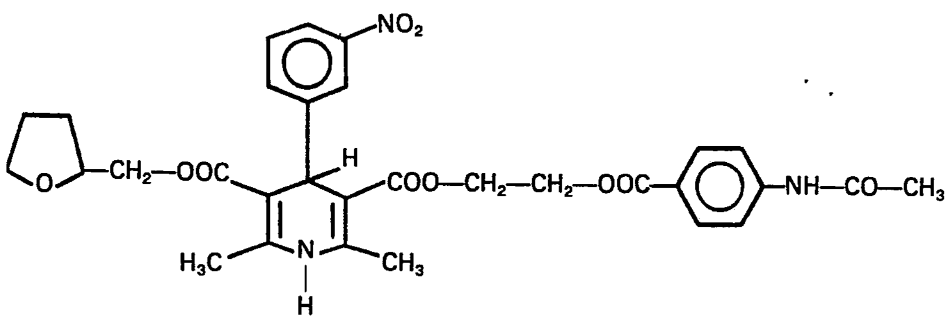

On chauffe à reflux pendant 8 heures une solution de 13,27 g (0,03 mol) de 2-(3-nitrobenzylidène)acétylacétate de 2-(4-acétylaminobenzoyloxy)éthyle et 5,58 g (0,03 mol) de 3-aminocrotonate de 2-tétrahydrofurfuryle dans 50 ml d'éthanol. Après décoloration avec charbon actif de la solution résultante, le solvant est évaporé pour obtenir ainsi le 2,6-diméthyl-4-(3-nitrophényl)-5-(2-tétrahydrofurfuryloxycarbonyl)-1,4-dihydropyridine-3-carboxylate de 2-(4-acétylaminobenzoyloxy)éthyle, sous la forme de cristaux jaunes écumeux, fondant à 82—92°C avec décomposition; le rendement est de 45% de la théorie.

| Analyse pour $C_{31}H_{33}N_3O_{10}$: | %C | %H | %N |
|---|---|---|---|
| Calculé | 61,28 | 5,47 | 6,92 |
| Trouvé | 58,69 | 5,35 | 6,83 |

Spectre I.R. (KBr) ν($cm^{-1}$): 3350, 2950, 2880, 1700, 1600, 1530, 1490, 1350, 1270, 1210, 1090, 1010, 855, 760, 700.

Spectre R.M.N. (δ, $CDCl_3$) p.p.m.: 8,2 à 7 (9H, m); 6,5 (1H, s); 5,1 (1H, s); 4,4 (4H, s); 4,1 à 3,6 (3H + 2H, s + m); 2,35 (6H, s); 2,2 (3H, s); 1,8 (4H, m).

Exemple 48

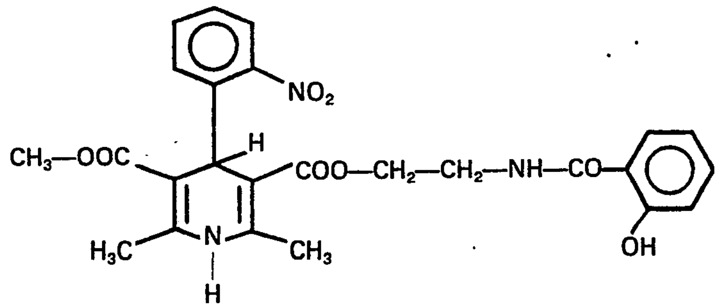

On chauffe à reflux pendant 8 heures une solution de 15 g (0,06 mol) de 3-aminocrotonate de 2-(N-salicylamido)éthyle et 14,15 g (0,06 mol) de 2-(2-nitrobenzylidène)acétylacétate de méthyle dans 60 ml d'éthanol. Après l'évaporation du solvant, addition 15 ml de méthanol à ébullition et refroidissement à −5°C, on obtient le 2,6-diméthyl-5-méthoxycarbonyl-4-(2-nitrophényl)-1,4-dihydropyridine-3-carboxylate de 2-(N-salicylamido)éthyle, sous forme de cristaux jaunes, fondant à 96—101°C (recristallisé dans éthanol); le rendement est de 77% de la théorie.

| Analyse pour $C_{25}H_{25}N_3O_8$: | %C | %H | %N |
|---|---|---|---|
| Calculé | 60,60 | 5,09 | 8,48 |
| Trouvé | 60,20 | 4,94 | 8,45 |

Spectre I.R. (KBr) ν($cm^{-1}$): 3380, 3080, 2960, 1710, 1650, 1600, 1540, 1500, 1360, 1310, 1215, 1090, 1020, 830, 750, 710

Spectre R.M.N. (δ, $CDCl_3$) p.p.m.: 12,2 (1H, s); 7,6 à 6,5 (10H, m); 5,7 (1H, s); 4,2 (2H, sl); 3,55 (2H + 3H, m + s); 2,3 (6H, d).

Exemple 49

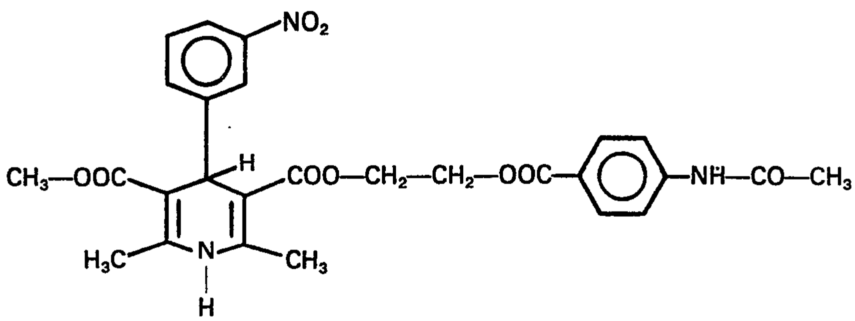

On chauffe à reflux pendant 8 heures une solution de 20 g (0,05 mol) de 2-(3-nitrobenzylidène)-acétylacétate de 2-(4-acétylaminobenzoyloxy)éthyle et 5,23 g (0,05 mol) de 3-aminocrotonate de méthyle dans 45 ml d'éthanol. Après le refroidissement du mélange de réaction à la température ambiante, on obtient le 2,6-diméthyl-5-méthoxycarbonyl-4-(3-nitrophényl)-1,4-dihydropyridine-3-carboxylate de 2-(4-acétylaminobenzoyloxy)éthyle, sous la forme de poudre jaune, fondant à 190—193°C; le rendement est de 94% de la théorie.

| Analyse pour $C_{27}H_{27}N_3O_9$: | %C | %H | %N |
|---|---|---|---|
| Calculé | 60,33 | 5,06 | 7,82 |
| Trouvé | 60,14 | 5,02 | 7,89 |

Spectre I.R. (KBr) ν($cm^{-1}$): 3360, 3240, 3100, 2960, 1720, 1700, 1660, 1530, 1480, 1350, 1270, 1210, 1110, 1090, 1000, 760, 700, 670.

Spectre R.M.N. (δ, DMSO-$d_6$) p.p.m.: 10,1 (1H, s); 8,9 (1H, s); 8 à 7,2 (8H, m); 5 (1H, s); 4,4 (4H, sl); 3,5 (3H, s); 2,35 (6H, s); 2,1 (3H, s).

Exemple 50

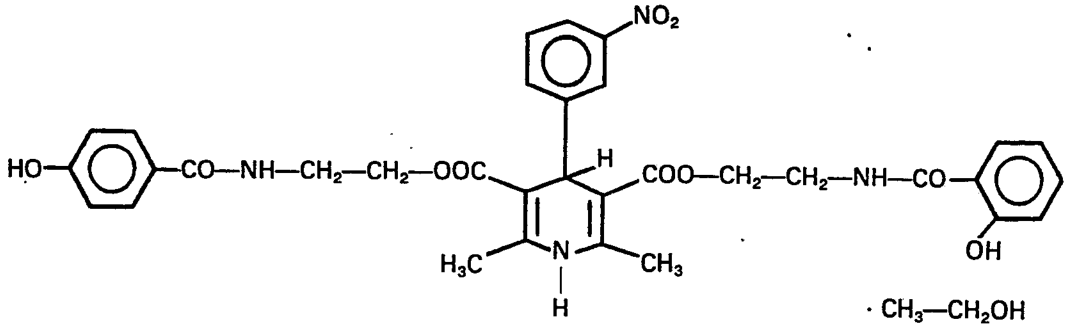

On chauffe à reflux pendant 8 heures une solution de 15 g (0,04 mol) de 2-(3-nitrobenzylidène)-acétylacétate de 2-(N-salicylamido)éthyle et 9,95 g (0,04 mol) de 3-aminocrotonate de 2-[N-(4-hydroxybenzoyl)amino]éthyle dans 40 ml d'éthanol. Après décoloration avec charbon actif et l'évaporation du solvant, on obtient le 2,6-diméthyl-4-(3-nitrophényl)-1,4-dihydropyridine-5-carboxylate de 2-[N-(4-hydroxybenzoyl)amino)]éthyle-3-carboxylate de 2-(N-salicylamido)éthyle, avec une molécule d'éthanol, sous la forme de cristaux jaunes écumeux, fondant à 75—100°C avec décomposition; le rendement est de 78% de la théorie.

| Analyse pour $C_{33}H_{32}N_4O_{10}.C_2H_6O$: | %C | %H | %N |
|---|---|---|---|
| Calculé | 60,86 | 5,55 | 8,11 |
| Trouvé | 60,55 | 5,26 | 7,95 |

Spectre I.R. (KBr) ν($cm^{-1}$): 3360, 2960, 1700, 1650, 1495, 1350, 1210, 1120, 1040, 840, 750, 700.

Spectre R.M.N. (δ, DMSO-$d_6$) p.p.m.: 8,2 à 6,6 (17H, m); 5 (1H, s); 4,15 (4H, sl); 3,7 à 3,3 (6H, m); 2,3 (6H, s); 1,1 (3H, t).

Exemple 51

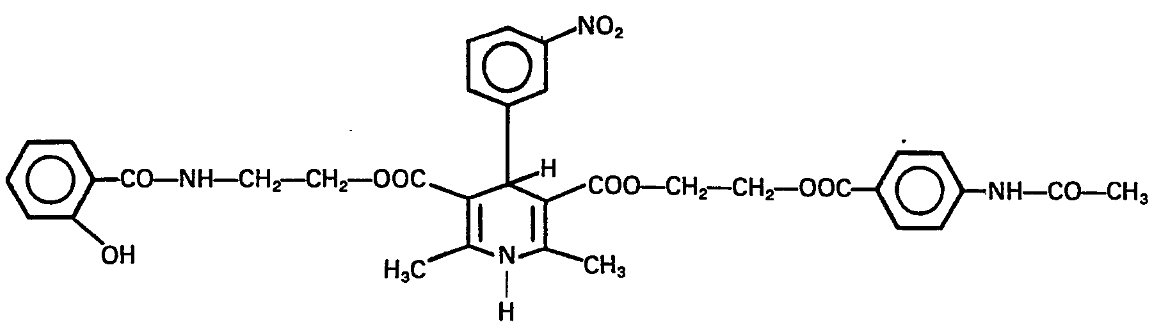

On chauffe à reflux pendant 12 heures une solution de 10 g (0,03 mol) de 2-(3-nitrobenzylidène)acétylacétate de 2-(4-acétylaminobenzoyloxy)éthyle et 9 g (0,03 mol) de 3-aminocrotonate de 2-(N-salicylamido)-éthyle dans 40 ml d'éthanol. Après l'évaporation de 20 ml du solvant et refroidissement à −5°C, obtient le 2,6-diméthyl-4-(3-nitrophényl)-1,4-dihydropyridine-5-carboxylate de 2-(N-salicylamido)éthyle-3-carboxylate de 2-(4-acétylaminobenzoyloxy)éthyle sous la forme de cristaux jaunes, fondant à 184—186°C (recristallisé dans éthanol aqueux 70%); le rendement est de 85% de la théorie.

| Analyse pour $C_{35}H_{34}N_4O_{11}$: | %C | %H | %N |
|---|---|---|---|
| Calculé | 61,22 | 4,99 | 8,16 |
| Trouvé | 61,11 | 5,08 | 8,01 |

Spectre I.R. (KBr) ν($cm^{-1}$): 3400, 2960, 1710, 1690, 1600, 1530, 1490, 1350, 1270, 1200, 1120, 1090, 860, 770, 740, 700.

Spectre R.M.N. (δ, DMSO-$d_6$) p.p.m.: 12,3 (1H, sl); 10,1 (1H, s); 9 à 8,7 (2H, m); 8,1 à 6,8 (12H, m); 5,05 (1H, s); 4,35 (6H, m); 3,55 (2H, sl); 2,35 (6H, s); 2,15 (3H, s).

Exemple 52

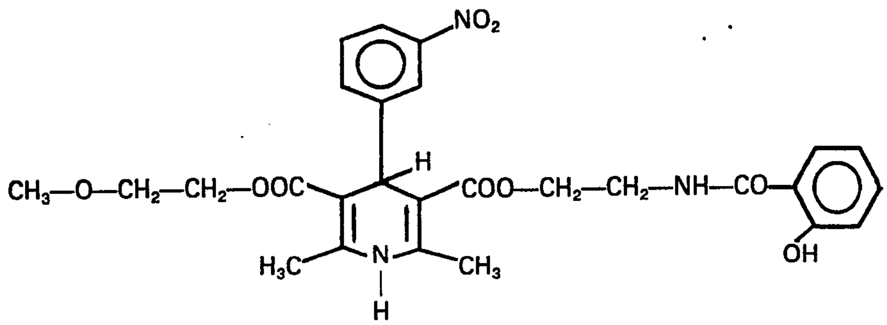

On chauffe à reflux pendant 10 heures une solution de 15 g (0,04 mol) de 2-(3-nitrobenzylidène)-acétylacétate de 2-(N-salicylamido)éthyle et 5,99 g (0,04 mol) de 3-aminocrotonate de 2-méthoxyéthyle dans 40 ml d'éthanol. Après décoloration avec charbon actif et évaporation du solvant, l'huile résultant se sèche sous vide dans dessicateur avec $CaCl_2$ pour obtenir ainsi le 2,6-diméthyl-5-(2-méthoxyéthoxycarbonyl)-4-(3-nitrophényl)-1,4-dihydropyridine-3-carboxylate de 2-(N-salicylamido)éthyle sous la forme de cristaux jaunes écumeux, fondant à 69—80°C; le rendement est de 70% de la théorie.

| Analyse pour $C_{27}H_{29}N_3O_0$: | %C | %H | %N |
|---|---|---|---|
| Calculé | 60,11 | 5,42 | 7,79 |
| Trouvé | 60,30 | 5,22 | 7,58 |

Spectre I.R. (KBr) ν(cm$^{-1}$): 3370, 3100, 2950, 1700, 1650, 1530, 1490, 1350, 1210, 1115, 1090, 1020, 780, 750, 700.

Spectre R.M.N. (δ, $CDCl_3$) p.p.m.: 8,1 à 6,5 (11H, m); 5,1 (1H s); 4,2 (4H, td); 3,6 (4H, td); 3,3 (3H, s); 2,35 (6H, s).

**Revendications**

1. Esters de 1,4-dihydropyridine et leurs sels, caractérisés par le fait qu'ils répondent à la formule:

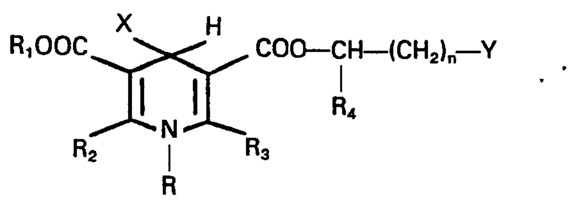

(I)

dans laquelle

R désigne un atome d'hydrogène, ou un radical alkyle à chaîne droite ou ramifiée en $C_1$ à $C_4$;

$R_2$ et $R_3$ représentent chacun un atome d'hydrogène ou un radical alkyle en $C_1$ à $C_4$;

$R_4$ désigne un atome d'hydrogène ou un radical alkyle à chaîne droite en $C_1$ ou $C_2$;

*n* est un nombre égal à 0, 1, 2 ou 3;

X désigne un radical phényle qui porte, le cas échéant, un à trois substituants nitro, alkyle en $C_1$ à $C_4$ ou alkoxy en $C_1$ à $C_3$, ou halogène;

$R_1$ désigne un radical alkyle en $C_1$ à $C_4$ ou alcényle en $C_2$ à $C_4$ à chaîne droite ou ramifiée, ou un groupe de formules

$$—W—O—Z \quad \text{ou} \quad —W—S—Z$$

dans lesquelles W désigne un groupe alkylénique à chaîne droite ou ramifiée en $C_1$ à $C_3$, et Z désigne un radical alkyle à chaîne droite ou ramifiée en $C_1$ à $C_4$, ou un groupe alicyclique contenant 5 ou 6 atomes de carbone, le cas échéant substitué par un ou plusieurs groupes méthyle, ou les groupe de formule

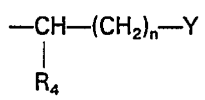

à l'exception du cas où Y représente le radical 2-tétrahydrofurfuryle; et

Y désigne le radical dérivé des amides N-substituées des acides nicotinique, salicylique et 4-hydroxy-benzoïque ou des pipérazines monoacylées ou sulfonylées N-substituées de formule:

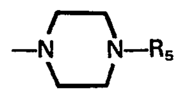

dans laquelle $R_5$ désigne un radical acyle choisi parmi les groupes acétyle, 2-furoyle, 2-thiophène-carbonyle et cinnamoyle, ou un radical benzène sulfonyle le cas échéant substitué par un ou plusieurs alkyle en $C_1$ à $C_2$, un ou plusieurs alkoxy en $C_1$ à $C_2$, un ou plusieurs trifluorométhyle, un ou plusieurs atomes d'halogène ou un radical acétylamine; ou un group de formule

$$—OCO—R_6 \quad \text{ou} \quad —OR_6$$

dans laqeulle $R_6$ désigne un radical acétylaminophényle, ou encore un groupe 2-tétrahydrofurfuryle ou N-(4-benzoyl-pipéridinyle).

2. Composés de formule (I) selon la revendication 1, caractérisés par le fait que

R désigne un atome d'hydrogène;

$R_2$ et $R_3$ désignent chacun un groupe méthyle;

$R_4$ désigne un atome d'hydrogène;

X désigne un groupe phényle portant un ou deux substituants, identiques ou différents, choisis parmi les groupes nitro, méthoxy, ou chloro;

*n* désigne un nombre égal à 0, 1 ou 2;

$R_1$ désigne un groupe alkyle en $C_1$ à $C_4$, alkoxyalkyle ayant 1 ou 2 atomes de carbone dans la partie alkylique et 1 à 3 atomes de carbone dans la partie alkoxylique, un groupe alkylthioalkyle avec 1, 2 ou 3 atomes de carbone dans les parties alkylique et thioalkylique, ou le groupe 3,3,5-triméthylcyclohéxyle ou allyle, ou enfin le groupe

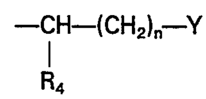

à l'exception du cas où Y représente le radical 2-tétrahydrofurfuryle; et

Y désigne le radical dérivé des amides N-substituées des acides nicotinique, salicylique et 4-hydroxy-benzoïque, ou des pipérazines N-substituées de formule:

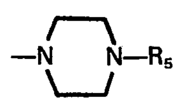

dans laquelle $R_5$ désigne les radicaux 2-furoyle, cinnamoyle ou benzènesulfonyle substitué ou non par un radical méthoxy ou chloro, ou un groupe de formule $—OCOR_6$ ou $—OR_6$ dans laquelle $R_6$ désigne la radical 4-acétylaminophényle, ou encore un groupe 2-tétrahydrofurfuryle ou N-(4-benzoyl-pipéridinyle).

3. Procédé de préparation d'esters de 1,4-dihydropyridines de formule:

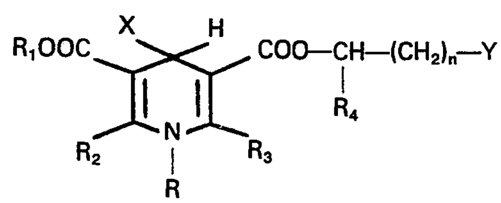

(I)

selon la revendication 1, et leurs sels, caractérisé par le fait qu'il consiste à faire réagir des esters d'acides β-cétocarboxyliques de formule:

$$R_2—CO—CH_2—COOR_1$$

dans laquelle $R_1$ et $R_2$ sont définis ci-dessus avec des amines de formule:

$$R—NH_2$$

dans laquelle R est défini ci-dessus le cas échéant après isolement des énamines éventuellement produites à partir des esters d'acides β-cétocarboxyliques et des amines, de formule:

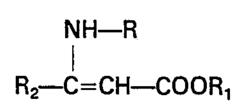

dans laquelle R, $R_1$ et $R_2$ sont définis ci-dessus avec les dérivés ylidéniques de formule:

$$R_4$$
$$|$$
$$COO—CH—(CH_2)_n—Y$$
$$|$$
$$X—CH=C—CO—R_3$$

dans laquelle X, $R_3$, $R_4$, n et Y sont définis ci-dessus, obtenus par réaction des aldéhydes de formule:

$$X—CHO$$

dans laquelle X est défini ci-dessus avec les esters d'acides β-cétocarboxyliques de formule:

$$R_3—CO—CH_2—COO—CH—(CH_2)_n—Y$$
$$|$$
$$R_4$$

dans laquelle $R_3$, $R_4$, n et Y sont définis ci-dessus et, le cas échéant, à préparer le sel des composés obtenus, avec un acide.

4. Procédé de préparation d'esters de 1,4-dihydropyridines de formule (I) selon la revendication 1, et leurs sels, caractérisé par le fait qu'il consiste à faire réagir des esters d'acides β-cétocarboxyliques de formule:

$$R_3—CO—CH_2COO—CH—(CH_2)_n—Y$$
$$|$$
$$R_4$$

dans laquelle $R_3$, $R_4$, n et Y sont définis ci-dessus avec des amines de formule:

$$R—NH_2$$

dans laquelle R est défini ci-dessus le cas échéant après isolement des énamines éventuellement produites à partir des esters d'acides β-cétocarboxyliques et des amines, de formule:

$$NH—R$$
$$|$$
$$R_3—C=CH—COO—CH—(CH_2)_n—Y$$
$$|$$
$$R_4$$

dans laquelle R, $R_3$, $R_4$, n et Y ont les définitions données ci-dessus avec des dérivés ylidéniques de formule:

$$COOR_1$$
$$|$$
$$X—CH=C—CO—R_2$$

dans laquelle X, $R_1$ et $R_2$ sont définis ci-dessus, obtenus par réaction des aldéhydes de formule:

$$X—CHO$$

dans laquelle X est défini ci-dessus avec les esters d'acides β-cétocarboxyliques de formule:

$$R_2—CO—CH_2COOR_1$$

dans laquelle $R_1$ et $R_2$ sont définis ci-dessus et, le cas échéant à préparer le sel des composés obtenus, avec un acide.

5. Procédé de préparation d'esters de 1,4-dihydropyridines de formule (I) selon la revendication 1, et leurs sels, caractérisé par le fait qu'il consiste à faire réagir des esters d'acides β-cétocarboxyliques de formule:

$$R_2—CO—CH_2COOR_1$$

dans laquelle $R_1$ et $R_2$ sont définis ci-dessus avec des énamines de formule:

$$\begin{array}{l} \quad NH-R \\ \quad | \\ R_3-C=CH-COO-CH-(CH_2)_n-Y \\ \qquad\qquad\qquad\qquad | \\ \qquad\qquad\qquad\qquad R_4 \end{array}$$

dans laquelle R, $R_3$, $R_4$, n et Y ont les définitions données ci-dessus avec des aldéhydes de formule:

$$X-CHO$$

dans laquelle X est défini ci-dessus, puis à préparer éventuellement le sel des composés obtenus, avec un acide.

6. Procédé de préparation d'esters de 1,4-dihydropyridines de formule (J) selon la revendication 1, et leurs sels, caractérisé par la fait qu'il consiste à faire réagir des esters d'acides β-cétocarboxyliques de formule:

$$\begin{array}{l} R_3-CO-CH_2-COO-CH-(CH_2)_n-Y \\ \qquad\qquad\qquad\qquad\qquad | \\ \qquad\qquad\qquad\qquad\qquad R_4 \end{array}$$

dans laquelle $R_3$, $R_4$, n et Y sont définis ci-dessus avec des énamines de formule:

$$\begin{array}{l} \quad NH-R \\ \quad | \\ R_2-C=CH-COOR_1 \end{array}$$

dans laquelle R, $R_1$ et $R_2$ sont définis ci-dessus et avec des aldéhydes de formule:

$$X-CHO$$

dans laquelle X est défini ci-dessus et à preparer, le cas échéant, le sel des composés obtenus, avec un acide.

7. Procédé de préparation d'esters de 1,4-dihydropyridines de formule:

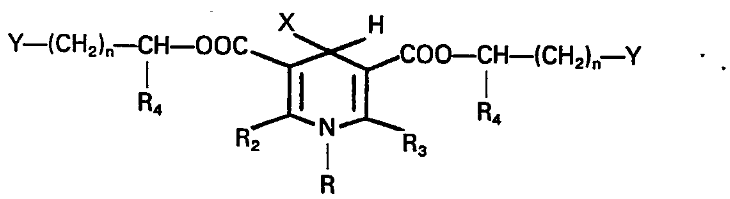

dans laquelle X, R, $R_2$, $R_3$, $R_4$, n et Y ont les définitions données dans la revendiction 1 et leurs sels, caractérisé par le fait qu'il consiste à faire réagir deux parties molaires d'esters d'acides β-cétocarboxyliques de formule:

$$\begin{array}{l} R_3-CO-CH_2-COO-CH-(CH_2)_n-Y \\ \qquad\qquad\qquad\qquad\qquad | \\ \qquad\qquad\qquad\qquad\qquad R_4 \end{array}$$

dans laquelle $R_3$, $R_4$, n et Y sont définis ci-dessus avec une partie molaire d'amine de formule:

$$R-NH_2$$

dans laquelle R est défini ci-dessus et avec une partie molaire d'un aldéhyde de formule:

$$X-CHO$$

dans laquelle X est défini ci-dessus et à préparer, le cas échéant, le sel des composés obtenus, avec un acide.

8. Médicament destiné au traitement préventif et curatif de troubles cardiovasculaire, caractérisé par le fait qu'il contient au moins un ester de 1,4-dihydropyridine selon la revendication 1.

9. Médicament suivant la revendication 8, caractérisé par le fait qu'il contient en outre des supports inertes non toxiques, acceptables du point de vue pharmaceutique.

**Patentansprüche**

1. 1,4-Dihydropyridinester und ihre Salze, gekennzeichnet durch die Formel:

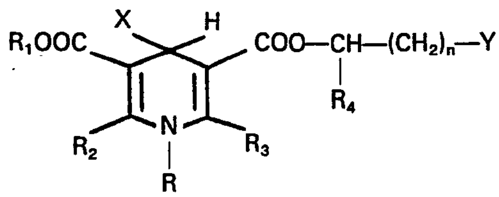

(I)

in der

R ein Wasserstoffatom oder ein Alkylradikal mit geradliniger oder verzweigter Kette in $C_1$ bis $C_4$ bezeichnet;

$R_2$ und $R_3$ je ein Wasserstoffatom oder ein Alkylradikal in $C_1$ bis $C_4$ darstellen;

$R_4$ beizeichnet ein Wasserstoffatom oder ein Alkylradikal mit geradliniger Kette in $C_1$ oder $C_2$;

*n* ist eine Zahl gleich 0, 1, 2 oder 3;

X bezeichnet ein Phenylradikal, das gegebenenfalls, ein bis drei Nitro-, Alkylsubstituenten in $C_1$ und $C_4$ oder Alkoxydsubstituenten in $C_1$ bis $C_3$ oder Halogensubstituenten enthält;

$R_1$ bezeichnet ein Alkylradikal in $C_1$ bis $C_4$ oder ein Alkenylradikal in $C_2$ bis $C_4$ mit geradliniger oder verzweigter Kette oder eine Gruppe mit den Formeln

$$-W-O-Z \text{ oder } -W-S-Z,$$

in der W eine alkylenische Gruppe mit geradliniger oder verzweigter Kette in $C_1$ bis $C_3$ bezeichnet und Z ein Alkylradikal mit geradliniger oder verzweigter Kette $C_1$ bis $C_4$ oder eine alkizylklische Gruppe, die 5 oder 6 Kohlenstoffatome enthält, gegebenenfalls ersetzt durch eine oder mehrere Methylgruppen, oder die Gruppe mit der Formel

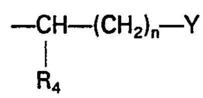

mit Ausnahme des Falles, in dem Y für das 2-Tetrahydrofurfuryl steht, und

Y das Radikal bezeichnet, gebildet aus N-substituierten Amiden der Nikotin-, Salicyl- und 4-Hydroxybenzosäuren oder der monoacylen Piperazine oder N-substituierten Sulfonylpeperazine mit der Formel:

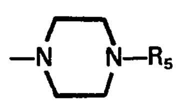

in der $R_5$ ein Acylradikal bezeichnet, ausgewählt aus den Acetyl-, 2-Furoyl-, 2-Thiophen-Karbonyl- und Cinnamoyl-Gruppen oder ein Sulphonylbenzolradikal, gegebenenfalls, substituiert durch ein oder mehrere Alkyle in $C_1$ bis $C_2$, ein oder mehrere Alkoxyde in $C_1$ bis $C_2$, ein oder mehrere Trifluoromethyle, ein oder mehrere Halogenatome oder ein Azetylaminradikal; oder eine Gruppe mit der Formel:

$$-OCO-R_6 \text{ oder } -OR_6$$

in der $R_6$ ein Azetylaminophenylradikal oder noch eine 2-Tetrahydrofurfuryl- oder N-(4-Benzoyl-Piperidinyl)-Gruppe bezeichnet.

2. Verbindung mit der Formel (I) nach Patentanspruch 1, dadurch gekennzeichnet, dass

R ein Wasserstoffatom bezeichnet;

$R_2$ und $R_3$ je eine Methylgruppe bezeichnen;

$R_4$ ein Wasserstoffatom bezeichnet;

X eine Phenylgruppe mit ein oder zwei gleichen Substituenten bezeichnet, ausgewählt aus den Nitro-, Methoxy- oder Chlorgruppen;

*n* bezeichnet eine Zahl gleich 0, 1 oder 2;

$R_1$ bezeichnet eine Alkylgruppe in $C_1$ bis $C_4$, eine Alkoxyalkylgruppe mit 1 oder 2 Kohlenstoffatomen im Alkylteil und 1 bis 3 Kohlenstoffatomen im alkoxylischen Teil, einer Alkylthioalkylgruppe mit 1, 2 oder 3 Kohlenstoffatomen im Alkyl- und Thioalkylteil, oder die 3,3,5-Trimethylzyklohexyl- oder Allylgruppe, oder schliesslich die Gruppe

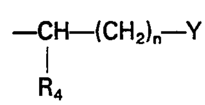

mit Ausnahme des Falles, im dem Y das 2-Tetrahyfurfuryl-Radikal darstellt; und

Y bezeichnet das Radikal, gebildet aus den N-substituierten Amiden der Nikotin-, Salicyl- und 4-Hydroxybenzosäuren oder N-substituierten Piperazinen mit der Formel:

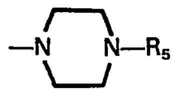

in dieser bezeichnet $R_5$ die 2-Furoyl-, Cinnamoyl- oder Benzensulfonyl-Radikale, substituiert oder nicht durch ein Methoxy- oder Chlorradikal, oder eine Gruppe mit der Formel $—OCOR_6$ oder $—OR_6$, in der $R_6$ das 4-Azetylaminophenylradikal oder aber eine 2-Tetrahydrofurfuryl- oder N-(4-Benzoyl-Piperidinyl)-Gruppe bezeichnet.

3. Verfahren zur Herstellung von 1,4-Dihydropropyridinestern mit der Formel:

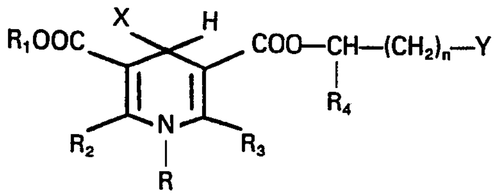

(I)

nach Patentanspruch 1, und deren Salze, dadurch gekennzeichnet, dass es darin besteht β-Zetokarboxylsäureester mit der Formel:

$$R_2—CO—CH_2—COOR_1$$

reagieren zu lassen, worin $R_1$ und $R_2$ oben definiert sind mit Aminen mit der Formel:

$$R—NH_2$$

darin ist R so wie oben definiert, gegebenenfalls nach Isolieren der eventuell erzeugten Enamine aus β-Zetokarboxylsäureestern und -aminen mit der Formel:

$NH—R$
|
$R_2—C{=}CH—COOR_1$

darin sind R, $R_1$ und $R_2$ so wie oben definiert mit Ylidderivaten mit der Formel:

$R_4$
|
$COO—CH—(CH_2)_n—Y$
|
$X—CH{=}C—CO—R_3$

darin sind X, $R_3$, $R_4$, n und Y so wie oben definiert, erhalten durch Reaktion von Aldehyden mit der Formel:

$$X—CHO$$

darin ist X so wie oben definiert mit β-Zetokarboxylsäureestern mit der Formel:

$R_3—CO—CH_2—COO—CH—(CH_2)_n—Y$
|
$R_4$

darin sind $R_3$, $R_4$, n und Y so wie oben definiert, und gegebenenfalls, Ansetzen des Salzes der erhaltenen Verbindung mit einer Säure.

4. Verfahren zur Herstellung von 1,4-Dihydropropyridinestern mit der Formel (I) nach Patentanspruch 1, und deren Salze, dadurch gekennzeichnet, dass es darin besteht β-Zetokarboxylsäureester mit der Formel:

$R_3—CO—CH_2—COO—CH—(CH_2)_n—Y$
|
$R_4$

worin R, $R_3$, $R_4$, n und Y so wie oben definiert sind, mit Aminen mit der Formel:

$$R—NH_2$$

worin R so wie oben definiert ist, gegebenenfalls nach Isolieren der eventuell erzeugten Enamine aus β-Zetokarboxylsäureestern und -aminen mit der Formel:

NH—R
|
$R_3$—C=CH—COO—CH—$(CH_2)_n$—Y
|
$R_4$

worin R, $R_3$, $R_4$, n und Y so wie oben definiert sind, mit Ylidenderivaten mit der Formel:

$COOR_1$
|
X—CH=C—CO—$R_2$

worin X, $R_1$ und $R_2$ so wie oben definiert sind, erhalten durch die Reaktion von Aldehyden mit der Formel:

$$X—CHO$$

worin X so wie oben definiert ist, mit den β-Zetokarboxylsäureestern mit der Formel:

$$R_2—CO—CH_2—COOR_1$$

worin $R_1$ und $R_2$ so wie oben definiert sind, zur Reaktion zu bringen und, gegebenenfalls, Ansetzen des Salzes der erhaltenen Verbindung mit einer Säure.

5. Verfahren zur Herstellung von 1,4-Dihydropyridinestern mit der Formel (I) nach Patentanspruch 1, und deren Salze, dadurch gekennzeichnet, dass es darin besteht β-Zetokarboxylsäureester mit der Formel:

$$R_2—CO—CH_2—COOR_1$$

worin $R_1$ und $R_2$ so wie oben definiert sind, mit Enaminen mit der Formel:

NH—R
|
$R_3$—C=CH—COO—CH—$(CH_2)_n$—Y
|
$R_4$

worin R, $R_3$, $R_4$, n und Y so wie oben definiert sind, mit Aldehyden mit der Formel:

$$X—CHO$$

worin X so wie oben definiert ist, zur Reaktion zu bringen und dann eventuelles Ansetzen des Salzes der erhaltenen Verbindung mit einer Säure.

6. Verfahren zur Herstellung von 1,4-Dihydropyridinestern mit der Formel (I) nach Patentanspruch 1, und deren Salze, dadurch gekennzeichnet, dass es darin besteht β-Zetokarboxylsäureester mit der Formel:

$R_3$—CO—$CH_2$—COO—CH—$(CH_2)_n$—Y
|
$R_4$

worin $R_3$, $R_4$, n und Y so wie oben definiert sind, mit Enaminen mit der Formel:

NH—R
|
$R_2$—C=CH—$COOR_1$

worin R, $R_1$ und $R_2$ so wie oben definiert sind, mit Aldehyden mit der Formel:

$$X—CHO$$

worin X so wie oben definiert ist, zur Reaktion zu bringen und Ansetzen des Salzes der erhaltenen Verbindung mit einer Säure.

7. Verfahren zur Herstellung von 1,4-Dihydropyridinestern mit der Formel:

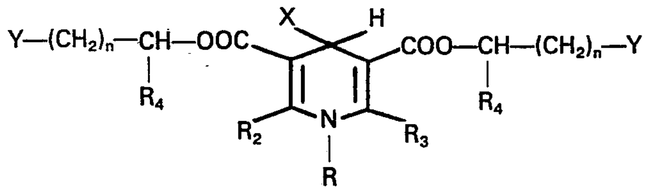

worin X, R, $R_2$, $R_3$, $R_4$ n und Y die im Patentanspruch 1 angegebenen Definitionen haben, und deren Salze, dadurch gekennzeichnet, dass es darin besteht, dass man zwei Molteile von β-Zetokarboxylsäureester mit der Formel:

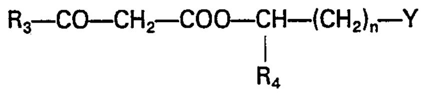

worin $R_3$, $R_4$, n und Y so wie oben definiert sind, mit einem Aminmolteil mit der Formel:

$$R—NH_2$$

worin R so wie oben definiert ist, und mit einem Aldehydmolteil mit der Formel:

$$X—CHO$$

worin X so wie oben definiert ist, zur Reaktion bringt, und Ansetzen des Salzes der erhaltenen Verbindung mit einer Säure.

8. Medikament bestimmt zur vorbeugenden und heilenden Behandlung von kardiovaskulären Beschwerden, dadurch gekennzeichnet, dass es mindestens einen 1,4-Dihydropyridinester nach Patentanspruch 1 enthält.

9. Medikament nach Patentanspruch 8, dadurch gekennzeichnet, dass es unter anderem inerte, nichttoxische, vom pharmazeutischen Standpunkt aus akzeptable Träger enthält.

**Claims**

1. 1,4-Dihydropyridine esters and salts thereof, characterized in that they have the formula

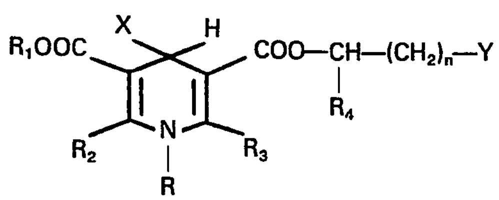

(I)

in which

R is a hydrogen atom or a linear or branched $C_1$ to $C_4$ alkyl radical;

$R_2$ and $R_3$ are each a hydrogen atom or a $C_1$ to $C_4$ alkyl radical;

$R_4$ is a hydrogen atom or a linear $C_1$ or $C_2$ alkyl radical;

*n* is a number equal to 0, 1, 2 or 3;

X is a phenyl radical which may or may not carry one to three nitro, $C_1$ to $C_4$ alkyl, $C_1$ to $C_3$ alkoxy or halogen substituents;

$R_1$ is a linear or branched $C_1$ to $C_4$ alkyl or $C_2$ to $C_4$ alkenyl radical; a group of the formula

$$—W—O—Z \quad \text{or} \quad —W—S—Z$$

in which W is a linear or branched $C_1$ to $C_3$ alkylene group and Z is a linear or branched $C_1$ to $C_4$ alkyl radical; an alicyclic group containing 5 to 6 carbon atoms which is unsubstituted or substituted by one or more methyl groups; or the group of the formula

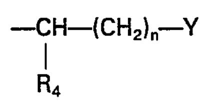

with the exception of the case where Y is the 2-tetrahydrofurfuryl radical; and

Y is the radical derived from the N-substituted amides of nicotinic, salicylic and 4-hydroxybenzoic acids or from the N-substituted monoacylated or sulphonylated piperazines of the formula

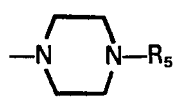

in which $R_5$ is an acyl radical selected from acetyl, 2-furoyl, 2-thiophenecarbonyl and cinnamoyl groups or a benzenesulphonyl radical which is unsubstituted or substituted by one or more $C_1$ or $C_2$ alkyl radicals, one or more $C_1$ or $C_2$ alkoxy radicals, one or more trifluoromethyl radicals, one or more halogen atomes or an acetylamine radical; a group of the formula

$$—OCO—R_6 \quad \text{or} \quad —OR_6$$

in which $R_6$ is an acetylaminophenyl radical; or a 2-tetrahydrofurfuryl or N-(4-benzoylpiperidinyl) group.

2. Compounds of formula (I) according to Claim 1, characterized in that

R is a hydrogen atom;

$R_2$ and $R_3$ are each a methyl group;

$R_4$ is a hydrogen atom;

X is a phenyl group carrying one or two identical or different substituents selected from nitro, methoxy and chloro groups;

*n* is a number equal to 0, 1 or 2;

$R_1$ is a $C_1$ to $C_4$ alkyl group, an alkoxyalkyl group having 1 or 2 carbon atoms in the alkyl moiety and 1 to 3 carbon atoms in the alkoxy moiety, an alkylthioalkyl group with 1, 2 or 3 carbon atoms in the alkyl and thioalkyl moieties, the 3,3,5-trimethylcyclohexyl or allyl group or, finally, the group

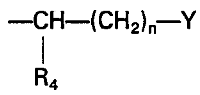

with the exception of the case where Y is the 2-tetrahydrofurfuryl radical; and

Y is the radical derived from the N-substituted amides of nicotinic, salicylic and 4-hydroxybenzoic acids or from the N-substituted piperazines of the formula

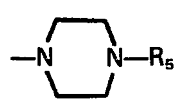

in which $R_5$ is the 2-furoyl or cinnamoyl radical or a benzenesulphonyl radical which is unsubstituted or substituted by a methoxy or chloro radical; a group of the formula $—OCOR_6$ or $—OR_6$ in which $R_6$ is the 4-acetylaminophenyl radical; or a 2-tetrahydrofurfuryl or N-(4-benzoylpiperidinyl) group.

3. A method of preparing 1,4-dihydropyridine esters of the formula

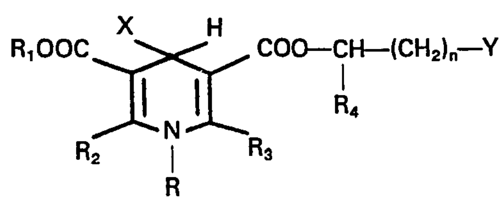

(I)

according to Claim 1, and salts thereof, characterized in that it consists in reacting β-ketocarboxylic acid esters of the formula

$$R_2—CO—CH_2—COOR_1$$

in which $R_1$ and $R_2$ are as defined above, with amines of the formula

$$R—NH_2$$

in which R is as defined above, and in reacting the resulting products, if appropriate after isolation of the enamines which may have been produced from the β-ketocarboxylic acid esters and the amines, of the formula

$$\begin{array}{l} \quad\;\; NH{-}R \\ \qquad | \\ R_2{-}C{=}CH{-}COOR_1 \end{array}$$

in which R, $R_1$ and $R_2$ are as defined above, with the ylidene derivatives of the formula

$$\begin{array}{l} \qquad\qquad\qquad\quad R_4 \\ \qquad\qquad\qquad\quad\; | \\ \qquad\quad COO{-}CH{-}(CH_2)_n{-}Y \\ \qquad\qquad | \\ X{-}CH{=}C{-}CO{-}R_3 \end{array}$$

in which X, $R_3$, $R_4$, n and Y are as defined above, which are obtained by reacting the aldehydes of the formula

$$X{-}CHO$$

in which X is as defined above, with the β-ketocarboxylic acid esters of the formula

$$\begin{array}{l} R_3{-}CO{-}CH_2{-}COO{-}CH{-}(CH_2)_n{-}Y \\ \qquad\qquad\qquad\qquad\quad\; | \\ \qquad\qquad\qquad\qquad\quad R_4 \end{array}$$

in which $R_3$, $R_4$, n and Y are as defined above, and, if desired, in preparing the salt of the resulting compounds with an acid.

4. A method of preparing 1,4-dihydropyridine esters of formula (I) according to Claim 1, and salts thereof, characterized in that it consists in reacting β-ketocarboxylic acid esters of the formula

$$\begin{array}{l} R_3{-}CO{-}CH_2COO{-}CH{-}(CH_2)_n{-}Y \\ \qquad\qquad\qquad\qquad\; | \\ \qquad\qquad\qquad\qquad R_4 \end{array}$$

in which $R_3$, $R_4$, n and Y are as defined above, with amines of the formula

$$R{-}NH_2$$

in which R is as defined above, and in reacting the resulting products, if appropriate after isolation of the enamines which may have been produced from the β-ketocarboxylic acid esters and the amines, of the formula

$$\begin{array}{l} \quad\;\; NH{-}R \\ \qquad | \\ R_3{-}C{=}CH{-}COO{-}CH{-}(CH_2)_n{-}Y \\ \qquad\qquad\qquad\qquad\; | \\ \qquad\qquad\qquad\qquad R_4 \end{array}$$

in which R, $R_3$, $R_4$, n and Y are as defined above, with ylidene derivatives of the formula

$$\begin{array}{l} \qquad\quad COOR_1 \\ \qquad\qquad | \\ X{-}CH{=}C{-}CO{-}R_2 \end{array}$$

in which X, $R_1$ and $R_2$ are as defined above, which are obtained by reacting the aldehydes of the formula

$$X{-}CHO$$

in which X is as defined above, with the β-ketocarboxylic acid esters of the formula

$$R_2{-}CO{-}CH_2{-}COOR_1$$

in which $R_1$ and $R_2$ are as defined above, and, if desired, in preparing the salt of the resulting compounds with an acid.

5. A method of preparing 1,4-dihydropyridine esters of formula (I) according to Claim 1, and salts thereof, characterized in that it consists in reacting β-ketocarboxylic acid esters of the formula

$$R_2{-}CO{-}CH_2{-}COOR_1$$

in which $R_1$ and $R_2$ are as defined above, with enamines of the formula

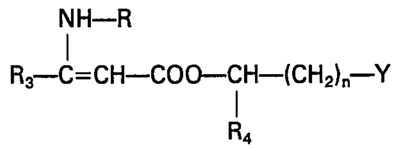

in which R, $R_3$, $R_4$, n and Y are as defined above, and with aldehydes of the formula

$$X—CHO$$

in which X is as defined above, and then, if desired, in preparing the salt of the resulting compounds with an acid.

6. A method of preparing 1,4-dihydropyridine esters of formula (I) according to Claim 1, and salts thereof, characterized in that it consists in reacting β-ketocarboxylic acid esters of the formula

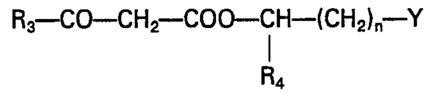

in which $R_3$, $R_4$, n and Y are as defined above, with enamines of the formula

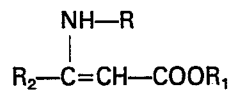

in which R, $R_1$ and $R_2$ are as defined above, and with aldehydes of the formula

$$X—CHO$$

in which X is as defined above, and, if desired, in preparing the salt of the resulting compound with an acid.

7. A method of preparing 1,4-dihydropyridine esters of the formula

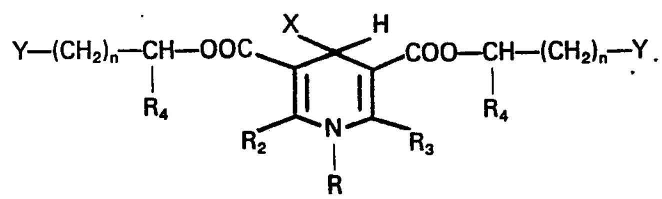

in which X, R, $R_2$, $R_3$, $R_4$, n and Y are as defined in Claim 1, and salts thereof, characterized in that it consists in reacting two molar parts of β-ketocarboxylic acid esters of the formula

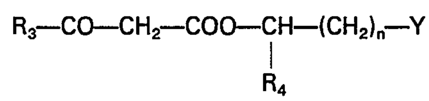

in which $R_3$, $R_4$, n and Y are as defined above, with one molar part of an amine of the formula

$$R—NH_2$$

in which R is as defined above, and with one molar part of an aldehyde of the formula

$$X—CHO$$

in which X is as defined above, and, if desired, in preparing the salt of the resulting compounds with an acid.

8. A drug for the preventive and curative treatment of cardiovascular disorders, characterized in that it contains at least one 1,4-dihydropyridine ester according to Claim 1.

9. A drug according to Claim 8, characterized in that it also contains pharmaceutically acceptable, non-toxic, inert excipients.